# EUROPEAN PATENT APPLICATION

(11) **EP 0 952 220 A2**
(43) Date of publication of application: **27.10.1999**
(21) Application number: 99200717.9
(22) Date of filing: 14.01.1994
(51) Int. Cl.: C12N 15/52, C12N 1/38, C12Q 1/02, C12Q 1/18, C12Q 1/68, C12P 1/04

(54) **Method to predict active growth of bacteria adhered to a surface**

(30) Priority: 15.01.1993 US 4971; 11.03.1993 US 29675
(62) Divisional of application: 94907825.7
(71) Applicant: REGENTS OF THE UNIVERSITY OF MINNESOTA, Minneapolis MN 55455 (US)
(72) Inventor: Bloomquist, Cynthia G., St. Louis Park, Minnesota 55426 (US); Douglas, William, Falcon Heights, Minnesota 55113 (US); Dunny, Gary, St. Paul, Minnesota 55116 (US); Liljemark, William, Plymouth, Minnesota 55447 (US); Reilly, Bernard, Minneapolis, Minnesota 55414 (US)
(74) Representative: Ottevangers, Sietse Ulbe

(57) **Abstract**

The present invention described provides a start microbial replication factor, as well as methods for assaying and using the start microbial replication factor. A start microbial replication factor can be used to stimulate the growth of bacteria in suspension or bound to a surface. Agents that inhibit or enhance a start microbial replication factor can also be identified. The present invention also describes processes to treat or control the growth of bacteria which are bound to surfaces. Both proliferation and metabolism of bacteria which are adhered to surfaces are independent of both time and nutrient supply. Growth of adherent bacteria is density dependent. The present invention also provides a process to predict when bacterial growth will exhibit rapid growth on a surface. The invention also provides strains of transformed bacteria and methods of using the bacteria strains to measure microbial growth in the presence of absence of a start microbial replication factor. The transformed bacteria have a bacterial reporter gene under the control of a promoter whose expression is coordinately regulated with cell growth.

### Annex to the description

SEQUENCE LISTING

(1) GENERAL INFORMATION:

(i) APPLICANT: Bloomquist, Cynthia G.
Douglas, William H.
Dunny, Gary M.
Liljemark, Wiliam F.
Reilly, Bernard E.

(ii) TITLE OF INVENTION: Composition and Process to Treat
or
Control Bacterial Infection

(iii) NUMBER OF SEQUENCES: 2

(iv) CORRESPONDENCE ADDRESS:
(A) ADDRESSEE: Merchant & Gould
(B) STREET: 3100 Norwest Center
(C) CITY: Minneapolis
(D) STATE: Minnesota
(E) COUNTRY: U.S.A.
(F) ZIP: 55402

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: US 08/029,675
(B) FILING DATE: 11-MAR-1993
(C) CLASSIFICATION:

(vii) PRIOR APPLICATION DATA:
(A) APPLICATION NUMBER: US 08/004,971
(B) FILING DATE: 15-JAN-1993
(C) CLASSIFICATION:

(viii) ATTORNEY/AGENT INFORMATION:
(A) NAME: Kowalchyk, Katherine M.
(B) REGISTRATION NUMBER: 36,848
(C) REFERENCE/DOCKET NUMBER: 600.289-US-01

(ix) TELECOMMUNICATION INFORMATION:
(A) TELEPHONE: 612-332-5300
(B) TELEFAX: 612-332-9081

(2) INFORMATION FOR SEQ ID NO:1:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

TCAGGAATTC GGCTAACTAC GTGCCAGCAG
30

(2) INFORMATION FOR SEQ ID NO:2:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:2:

GAGCGGATCC CCCGGGAACG TATTCAGC
28

## Description

The present invention generally relates to microbial replication factors and processes for inhibiting or enhancing growth of microorganisms. One process involves treating or controlling a bacterial infection caused by adherent bacteria attached to or on surfaces. Another aspect of this invention particularly relates to a process to treat or control infection of adherent bacteria on surfaces of teeth or internal implanted surfaces. Another aspect of this invention relates to a composition used to initiate or start active or rapid growth or replication of bacteria and novel strains of bacteria to assay the composition. Still another aspect of this invention relates to a method to control active or rapid growth of adherent bacteria using a composition which initiates or starts active growth of bacteria.

### Background of the Invention

Treatment and control of bacterial infection has been a principal focus of modern research. The introduction and widespread use of antibiotics has provided the medical practitioner with a variety of powerful tools to treat and control bacterial infection in patients. In spite of the vast amounts of research undertaken as well as readily available pharmaceutical agents to combat bacterial infection, the problems of treating and controlling bacterial infection are not solved. For example, the widespread use of antibiotics has led to the development of strains of pathogenic organisms which are resistant to most commonly used antibiotics. In addition, the increasing number of immune-compromised patients has created new challenges for the medicai practitioner. Furthermore, medical practitioners have long recognized that the presence of indwelling foreign devices, such as dental implants, artificial heart valves or catheters, predisposes a patient to bacterial infection and complicates the treatment and control of infection related to such foreign bodies.

It is certainly expected, however, that the implantation of devices will continue to increase as advances are made in both biomaterials and implantation methodologies. In spite of this expected increase in the use of indwelling biomaterials, effective methodologies and pharmacological agents useful to treat and control bacteria associated with the use of these materials are not currently available.

The control of bacterial infestation and growth on surfaces of is particularly problematic because bacterial infestation of such surfaces is not well understood. Even though bacteria are prone to infest the surface of nearly all accessible articles, little is known about control of such bacteria on these surfaces or about the factors contributing to the growth of such bacteria on such surfaces.

Mechanisms thought to be significant in development of bacterial biomass on accessible surfaces include (1) adherence of indigenous bacteria to specific receptors of enamel pellicular surfaces; and (2) growth or cell division of adherent bacteria. It is thought that growth of adherent bacteria is very slow.

It has been observed and reported that bacteria which are adhered to surfaces are significantly different from bacteria which are in a solution or which are not bound to a surface. For example, bacteria which are in solution are more susceptible to antibiotics when compared to bacteria which are adhered to surfaces. Thus, an understanding of the processes used by bacteria to grow on or adhere to surfaces is necessary to successfully treat or control bacteria infections related directly or indirectly to surfaces.

In addition, microbial growth factors important in stimulating growth in a population of cells or initiating growth in an individual cell have not been identified or well characterized. The identification of microbial growth factors from bacterial cells growing on surfaces or in suspension could be important tools for enhancing the growth of bacteria or inhibiting the growth of bacteria in suspension or on surfaces.

Clearly, there exists a need to understand and to predict bacterial growth on the surface of biomaterials as well as control the proliferation and metabolism of adhered bacteria associated with these materials. A need also exists to identify and characterize microbial growth factors so that bacterial growth on surfaces or suspension can be enhanced or inhibited.

### Summary of the Invention

The present invention provides a novel solution to the above identified problems currently associated with treating or controlling bacterial growth on surfaces because it has now been determined that bacterial growth on a surface of a material is density dependent. Unless an adherent bacterial population approaches a density associated with an enhanced proportion of actively growing bacteria to viable bacteria, bacterial growth is unsubstantial. Put another way, substantial proliferation and metabolism of bacteria which are adhered to surfaces cf biomaterials are independent of both time and nutrient supply.

Thus, one aspect of the present invention provides a process to predict when bacteria will exhibit active growth on a surface which includes the steps of determining a first population density of viable bacteria on the surface. Then comparing the first population density with a second population density having a stimulated or enhanced proportion of actively growing bacteria to viable bacteria in the second population, preferably at a density of about 400,000 bacteria/mm² to about 6,300,000 bacteria/mm². Active bacterial growth can be predicted when the first population density is greater than the second population. The method can preferably be used to measure the growth of bacteria on surfaces. The proportion of actively growing cells to viable cells in a population may be readily determined using the processes described in Example 1, below.

Still another aspect of this invention is a process to predict a rapid increase in the growth of bacteria on a surface which includes the steps of determining a population density of viable growing bacteria on the surface, determining a population of actively growing bacteria on the surface, computing a ratio of actively growing bacteria to viable bacteria on the surface, and predicting active bacterial growth on the surface when the ratio of actively growing bacteria to viable bacteria is between about 5 x 10⁻⁴:1 to about 20 x 10⁻²:1. Preferably, the ratio of actively growing bacteria to viable bacteria is between about 20 x 10⁻⁴:1 to 40 x 10⁻⁴:1.

The invention also includes a method for inhibiting bacterial growth on a surface by controlling bacterial growth so that the population density is maintained below the population density associated with an enhancement or stimulation of the proportion of growing cells to viable cells in the population (i.e., density dependent growth). The bacterial growth can be controlled by limiting the contact of bacteria with adherence promoting agents such as carbohydrates or by modifying the hydrophobicity of the surface.

In addition to the methods referred to above, the present invention includes compositions and methods for controlling or treating bacterial infections. Thus, this invention includes a composition containing a start microbial replication factor, SMR factor, which will initiate or start the processes of active growth of bacteria which are bound to surfaces and, when isolated, has a molecular weight less than 3000 as measured by size exclusion membrane filtration. The isolated SMR factor is also resistant to autoclaving, freezing and lyophilizing.

A composition containing SMR factor may be prepared by forming a culture of sanguis streptococci and isolating extracellular material from the culture matrix when the bacteria are in an active growth phase. Both suspension and agar cultures of sanguis streptococci are useful in the preparation of SMR factor. Preferred strains of bacteria include S. gordonii strain Challis ATCC Deposit No. 35105 (formerly S. sanguis) or E. coli 149 ATCC No. 55535.

The presence of SMR factor may be readily determined by many methods including contacting the SMR-containing material with bacteria deposited on a saliva-coated enamel chip, incubating the bacterial cell-coated chip, and determining active growth by incorporation of radiolabelled nucleotides by the bacterial cells according to the method described in Example 1.

Other methods for determining SMR composition include measuring an increase in the proportion of actively growing cells immobilized to a surface in the presence of SMR compared to a population of bacteria not treated with SMR. The cells can be gram positive or gram negative but are preferably S. gordonii Challis ATCC No. 35105. Transformed bacterial strains having an integrated bacterial reporter gene under control of a promoter whose expression is coordinately regulated with cell growth can also be used.

The SMR factor can also be used in a method to increase the proportion of actively growing cells to viable cells in a population, preferably to a ratio of about 5 x 10⁻⁴:1 to 20 x 10⁻²:1. A stimulation of microbial growth processes could be important in many industrial and diagnostic situations.

This invention provides a method of increasing the efficacy of antimicrobial agents by contacting bacteria with the antimicrobial agent and SMR factor in order to initiate active growth of the bacteria. The invention also provides a method for identifying agents that inhibit or enhance a SMR factor. Agents that inhibit SMR factor activity could be novel chemotherapeutic agents. Agents that enhance SMR factor activity could be other novel microbial growth factors.

The invention also provides for a genetic cassette and transformed bacteria with an integrated genetic cassette. A genetic cassette is comprised of a first DNA sequence coding for a bacterial reporter gene linked to the 3' end of a second DNA sequence that hybridizes to a gene or portion thereof whose expression is coordinately regulated with bacterial growth. The second DNA sequence functions to integrate the genetic cassette or portion thereof into the gene in the bacterial chromosome. Transformed bacterial strains have the genetic cassette integrated into the bacterial chromosome at a location under the control of a promoter whose expression is coordinately regulated with cell growth. The transformed bacterial strains can be used in a method to measure microbial growth in the presence or absence of SMR factor by monitoring the expression of the bacterial reporter gene product per cell per unit time.

### Brief Description of the Drawings

Figure 1A is a graphic representation of active growth of bacteria on in vivo dental implants as population approaches a density of the bacteria of about 2,500,000 - 3,200,000 [10^{6.4-6.5}] bacteria/mm².

Figure 1B is a graphic representation of the saturation of enamel pellicular binding sites which occurs during the first 2 through 8 hours on in vivo dental implants. Oral flora saturate these surfaces at a density of 200,000 - 630,000 [10^{5.3} - 10^{5.8}] per mm².

Figure 2 is a graphic representation of active growth of bacteria on in vivo dental implants in the presence of sucrose, glucose or water as a bacterial population approaches a density of about 2,500,000 - 3,200,000 [10^{6.4-6.5}] bacteria/mm².

Figure 3 is a graphic representation of a comparison of total viable bacteria in the mouth on enamel and on enamel treated with sodium octadecyl sulfate.

Figure 4 is a graphic representation of active bacterial growth on the surface of bovine enamel, VISIO-DISPERS light-cured composite dental restorative (ESPE-Premier, Norristown, PA) and SILUX restorative (3M, St. Paul, MN) as the population density on any of such surfaces approaches about 2,500,000 - 3,200,000 [10^{6.4-6.5}] bacteria/mm².

Figure 5A represents the immobilization of radio-labeled S. gordonii Challis strain ATCC Deposit No. 35105, (*) and Streptococcus parasanguis PW213(•) to maleic anhydride activated plates as a function of cell input per mm².

Figure 5B represents the binding of radiolabelled S gordonii Challis strain ATCC Deposit No. 35105 as a function of cell input per mm² to three surfaces: reacti-bind maleic anhydride activated polystyrene strip plates (□) (Pierce, Rockford, Ill); Immulon strip plates (◇) (Dynatech Labs) and Easy Wash ELISA strip wells (•) (Corning, NY).

Figure 6 represents the incorporation of [³H-methyl]-thymidine into S. gordonii Challis strain ATCC Deposit No. 35105 in the presence of isolated SMR factor (*) and control Todd Hewitt (TH) media (□) as a function of the time of incubation of SMR or TH. Arrow depicts time of harvest in the preferred in vitro bacterial replication assay.

Figure 7 represents the genetic and physical map of plasmid pMSP16Samy. This plasmid was constructed by ligation of a fragment of DNA homologous to an internal segment of a streptococcal 16s rRNA gene into pRQ200 as described herein.

Figure 8 represents the postulated structure of chromosomal rDNA of S. gordonii Challis containing a promoterless amylase gene fused to a 16s rRNA promoter (indicated by P) following integration of pMSP16Samy into the Challis chromosome by homologous recombination.

### Detailed Description

The present invention discloses processes to treat or control the growth of bacteria which are attached or adhered to surfaces of biomaterials. The present invention provides processes to determine under what conditions and at what periods of time bacteria will exhibit enhanced, stimulated, active, or rapid growth on a surface. Active growth of bacteria is directly related to population density. Density dependent growth is associated with a stimulation or enhancement of the proportion of actively growing cells in the viable cell population. The proportion of actively growing cells to the number of viable cells in a population can be determined by a number of methods, but is preferably measured by incorporation of a radiolabelled nucleotide into cellular DNA. The stimulation or enhancement of the proportion of growing cells to viable cells results in a ratio of growing to viable cells of about 5 x 10⁻⁴:1 to 20 x 10⁻²:1, preferably 10 x 10⁻⁴:1 to 40 x 10⁻⁴:1. Density dependent active growth can be achieved when a natural predominantly streptococcal population of bacteria having a size approaching 1-2 microns has a population density of approaching about 400,000 [10^{5.6}] bacteria/mm² to about 6,300,000 [10^{6.8}] bacteria/mm². Preferably, the population density approaches about 2,500,000 - 3,200,000 [10^{6.4-6.5}] bacteria/mm².

Furthermore, the present invention provides microbial replication factors which are agents or products that initiate or start growth or replication processes in bacteria. Variations and modifications of these microbial replication factors permit control over the conditions and periods of time when bacteria will exhibit active growth. Methods are provided for determining the activity of microbial replication factors on cells and for identifying agents that inhibit or enhance microbial replication factors. Methods are also provided for using the microbial replication factors to enhance or inhibit microbial growth on surfaces or in suspension. Novel bacterial strains can be utilized in a method to measure microbial growth in the presence or absence of microbial growth factors.

The invention also provides genetic cassettes and transformed bacterial cells. A genetic cassette comprises a first DNA sequence coding for a bacterial reporter gene linked to the 3' end of a second DNA sequence. The second DNA sequence provides for recombination and integration of the genetic cassette into the bacterial chromosome at a location downstream from a promoter whose expression is coordinately regulated with cell growth. Transformed cells have a genetic cassette including a bacterial reporter gene stably integrated into the bacterial chromosome under the control of a promoter whose expression is coordinately regulated with cell growth. The reporter gene functions as an indicator of the proportion of growing cells in the bacterial population. Methods also are provided for measuring microbial growth in the presence or absence of microbial replication factors.

As used in this document, the following terms have the following meanings.

Viable Bacteria means living bacteria which will grow in the presence of a suitable nutrient-containing media. Typically, an aliquot or sample containing bacteria is added to a nutrient-rich agar medium and the bacteria in the aliquot or sample are grown. The number of bacteria contained in the sample may be readily determined by counting the number of colony forming units which grow on the agar. The number of viable bacteria, however, does not provide any information relating to a specific state of growth that such bacteria may be in at a given time. Thus, viable bacteria include dormant, latent, and actively growing and dying bacteria.

Actively Growing Bacteria means bacterial cells which are in a relatively brief or short period of time in which bacterial cells enter a period of active or rapid proliferation, metabolism, growth and replication compared to the rate of proliferation, metabolism, growth and replication observed for dormant, latent or slowly growing cells. Actively growing cells can be measured by looking at an increase in number of viable cells per unit of time when growing in liquid culture or suspension. When bacteria are in the exponential phase in liquid culture, a doubling time can be calculated based on the change in the numbers of viable cells per unit of time. An increase in the proportion of actively growing to viable cells in a population can be determined by measuring the proportion of cells actively synthesizing DNA or RNA or polypeptide. Total cell mass, polypeptide, RNA and DNA are functions of growth rate. See Physiology of the Bacterial Cell: A Molecular Approach, eds. F.C. Neidhardt et al. (1990) Sinauer Associates, Inc. at pages 199-202, 216-220. The proportion of actively growing cells can be measured by measuring the amount of cellular DNA synthesis with the labelling technique as described in Example 1.

Pathogenic Bacteria means any genera, strain or type of bacteria which will have a detrimental clinical impact on a patient where the detrimental effect is due to undesired bacterial growth or to the presence of undesired bacterial metabolites. Pathogenic bacteria are known to those of skill in the art and have been described in reference textbooks such as Medical Microbiology: An Introduction to Infectious Diseases, 2d Edition, John C. Sherris (editor) (1990) Elsivier Publishing Co., Inc., New York, NY. It is understood that the pathogenicity of a specific genera, strain or type of bacteria will vary from patient to patient. One of ordinary skill in the art, however, may readily determine what specific bacteria detrimentally impact a specific patient without having to undergo undue testing or experimentation. It is also understood that not all bacteria will detrimentally affect a patient and that the presence of known, relatively benign or innocuous bacteria (i.e., normal flora) on biomaterial surfaces is not considered to be detrimental and may be beneficial to a patient.

Opportunistic Bacteria are those microorganisms that are usually considered non-virulent or of limited virulence but that can cause disease or infection if they reach protected areas of the body or if the general host defense mechanisms are compromised. Opportunistic bacterial infections are known to those of skill in the art and have been described in textbooks such as Medical Microbiology, cited supra.

Population Density means the number of viable bacteria which are adhered or attached to a specific area of a surface or in a known volume of a liquid. The population density of such bacteria may be determined using well known culturing, plate counting methods and vital stains.

Biomaterial means articles having surfaces where viable bacteria may adhere, attach or stick. Suitable biomaterials include a variety of materials such as resins, restoratives, composites, metals or ceramics. Surfaces includes the exposed surfaces of materials which may contact bacteria contained in fluids.

Adherence Promoting Agents means materials, substances or compositions which promote or enhance the ability of bacteria to adhere, attach or stick to a surface.

Adherence Suppressing Agents means materials, substances or compositions which may be used to modify the surface properties of a material in order to suppress or inhibit the ability of bacteria to adhere, attach or stick to a biomaterial surface.

Start Microbial Replication Factor, SMR factor, means any agent, agents or a combination of agents which will enhance, stimulate, initiate or start bacterial cells to become actively growing cells. When SMR factor activity is examined on a population of bacterial cells, an increase in the proportion of actively growing cells to viable cells is seen, preferably with a ratio of actively growing cells to viable cells in the population of about 5 x 10⁻⁴:1 to 20 x 10⁻²:1. The term "actively growing" includes bacterial cell division as well as increased cellular metabolism, production of DNA and RNA or other biological responses which are associated with an active growth period of bacterial cells. It is understood that a variety of extraneous factors such as type of media, concentration, ionic strength, presence of ions, reduction-oxidation potential, viscosity, conduction capability, and other biological agents will affect the observed activity of SMR factor.

Dynamic Contact Angle means a measurement of the interaction of a surface or modified surface with water using a Wilhelmy balance according to the methodology set out by the manufacturer (the Wilhelmy balance is commercially available form Cahn Instruments, Model DCA-332, Cerritos, CA). The relative hydrophobicity of a surface or modified surface may be determined by this method.

Immobilized Bacteria means bacteria which are linker attached to a surface. Immobilization can include binding of bacteria to a surface using chemical or biological agents such as maleic anhydride or support bound antibodies. Adherent bacteria means bacteria attached to compatible surfaces using endogenous adhesins. Bound bacteria means bacteria associated with surfaces.

Bacterial Reporter Gene Product means a polypeptide which is preferably not normally present in a bacterial host cell whether actively growing or not and which can serve as an indicator of actively growing cells. The gene product has a structural or functional characteristic that can be measured as a function of time or growth.

Genetic Cassette means a combination of at least two different DNA sequences, preferably having different functions, to form a unit of genetic information.

Stably Integrated means that the genetic cassette is linked to the bacterial chromosome so that it is replicated and transmitted to progeny bacteria along with the bacterial chromosome. Integrated genetic cassettes have a very low frequency of reversion relative to genetically engineered plasmids, preferably about 10⁻⁵ to 10⁻⁹.

A Second DNA Sequence that hybridizes to a gene or portion thereof is one that shares sufficient sequence identity (preferably 90-100%) with a complementary region of the bacterial chromosome to form a stable, double-stranded hybrid DNA molecule when the hybridizing sequence is introduced into the bacterium by transformation. Hybrid molecules formed in vivo between cloned DNA sequences (preferably 500-5000 base pairs in length) can be used to integrate genetic cassettes into the bacterial chromosome at specific locations.

### Methods of Predicting Active Growth of Bacteria on a Surface

The present invention discloses that the proliferation and metabolism of bacteria which are bound to surfaces are independent of both time and nutrient supply and that such adhered bacteria will not significantly proliferate or metabolize unless the bacterial population approaches a population density associated with density dependent growth on natural surfaces.

In view of the fact that bacteria are found in essentially every part of the natural world and that when bacteria are present they inevitably attempt to adhere to accessible surfaces, methods and processes for controlling such bacteria have a wide range of applicability. Specifically, surfaces which are accessible to bacteria may include dental biomaterials, indwelling medical devices, other dental and medical instruments which come in contact with fluids such as sera, body fluids, other polypeptide-, glycoprotein- or carbohydrate-containing fluids as well as free-living bacteria. Surfaces found in the home, work and industrial environments are susceptible to adherence of bacteria.

The examination of biomaterials found in the mouth or oral cavity serves as a readily available model to study the behavior and properties of bacteria adhered to surfaces. Surfaces contained in the oral cavity are readily accessible and the oral cavity includes a wide range of bacterial genera and species which have been found to be detrimental in other protected areas of the body. For example, it has been reported that certain bacteria which are present in the oral cavity are capable of opportunistically infecting the endocardial surface of the heart which normally is a protected area and is usually sterile, i.e. is not typically contaminated by or with bacteria. This type of endocardial infection is especially problematic for patient's implanted with prosthetic heart valves. In view of the presence of the multiple bacteria genera and strains throughout the mucosal surfaces of the body as well as generally related environments, it is expected that methods and processes used to evaluate, treat and control bacteria adhered to surfaces in the oral cavity will readily transfer to the evaluation, treatment and control of bacteria adhered to surfaces in other parts of the body or the environment.

Furthermore, it is also expected that surfaces accessible to bacteria which contact sera or other body fluids also exhibit density dependent growth phenomena similar to the behavior of bacteria found in the oral cavity. Additionally, the methods and processes disclosed in this specification will be adaptable to evaluate, treat and control bacteria adhered to surfaces outside of either the oral cavity or body.

An important factor to consider in the development of methods and processes which impact on bacteria is the need to selectively target pathogenic or opportunistic bacteria without detrimentally affecting benign or beneficial bacteria associated with normal flora. The efficacy of antimicrobial agents may be significantly enhanced by contacting pathogenic bacteria with both a specific antimicrobial agent and start microbial replication (SMR) factor because it is well known that bacteria are particularly susceptible to antimicrobial agents when they are in a period of active growth but may be unaffected by antimicrobial agents when they are in a dormant or latent growth period.

One method for targeting growth of pathogenic or opportunistic bacteria is to contact bound bacteria with a specific antimicrobial agent in the presence of a start microbial replication factor. Without being meant to limit the invention, it is thought that adherent bacteria are nonresistant to antimicrobial agents, in part due to slow growth rates. Gustina et al., Biomaterials, 8:423 (1987) and Brown et al., Antimicrobial. Chemotherapy, 22:777 (1988). Growth rate is known to be the primary modulator of antibiotic action. Pathogenic bacteria can preferably be targeted by contacting the bacteria with an antimicrobial agent specific for the type of pathogenic or opportunistic bacteria and a microbial replication factor as described herein. While not meant to limit the invention in any way, it is believed that the stimulation of active growth would increase the proportion of pathogenic or opportunistic bacteria that are susceptible to the antimicrobial agent. Antimicrobial agents specific for pathogens and opportunistic bacteria are know to those of skill in the art and are described in Internal Medicine, 4th Ed., Editor in Chief Stein, Mosby-Year Book Inc., St. Louis, Missouri or Wilson et al., Harrison's Principles of Internal Medicine, 12th Ed., Vol. 1, Eds., McGraw-Hill, Inc., New York, NY.

Alternatively, it may not be necessary to take into account the presence of benign bacteria or normal flora in selecting an antimicrobial agent as certain protected areas of the body are considered sterile, i.e. not typically contaminated by or with bacteria. These protected areas of the body include the interior of the eye, the pericardial sac including the heart, internal organs, bones and muscles, and the brain. For controlling pathogenic or opportunistic infection in these protected areas of the body, an antimicrobial agent including a broad spectrum agent can be administered with a microbial replication factor.

Methods of the invention also include predicting when bacteria bound to a surface will exhibit active growth. The method involves determining a density at which the proportion of growing cells to viable cells in the population is increased to about 5 x 10⁻⁴:1 to about 20 x 10⁻²:1. An enhancement or stimulation of actively growing cells in a population of bacteria is density dependent. Once this density is determined, one can predict when a population of bound bacteria will exhibit active growth. The proportion of actively growing cells to viable cells in populations treated with various agents such as microbial replication factors can be compared.

The determination of population density of viable bacteria bound to surfaces may be accomplished using a variety of methods and processes. For example, the bacteria may be removed from a surface, appropriately diluted, placed on suitable media, incubated and counted using well known methodology. Alternative measurements of determining population densities are known to those of skill in the art. This allows the determination of viable bacteria population densities which is the most accurate dental plaque population density measurement to date.

A method for determining the proportion of actively growing cells in a population contacts an aliquot of a sample concurrently measured for viable counts per measured area, with an excess of labeled nucleoside. Typically, the length of contact time is less than the minimum cell division time. Incubating bacteria in the presence of the labeled nucleoside will result in incorporation of the label into the DNA (and/or RNA) of those viable cells which are actively growing. The amount of incorporated labeled nucleotide may then be used to determine the number or portion of actively growing bacteria compared to the viable bacteria in the sample. The ratio of active growth based on viable count can be compared between samples of different viable cell surface densities.

The labeled nucleotide may be selected from suitable purine and pyrimidine derivatives which have been modified to contain an appropriate label. Suitable labels may include radioactive ligand as well as dyes or other labels known to the skilled practitioner. A preferred labeled nucleotide is a mixture of [methyl³H]-thymidine and [C¹⁴]-adenine.

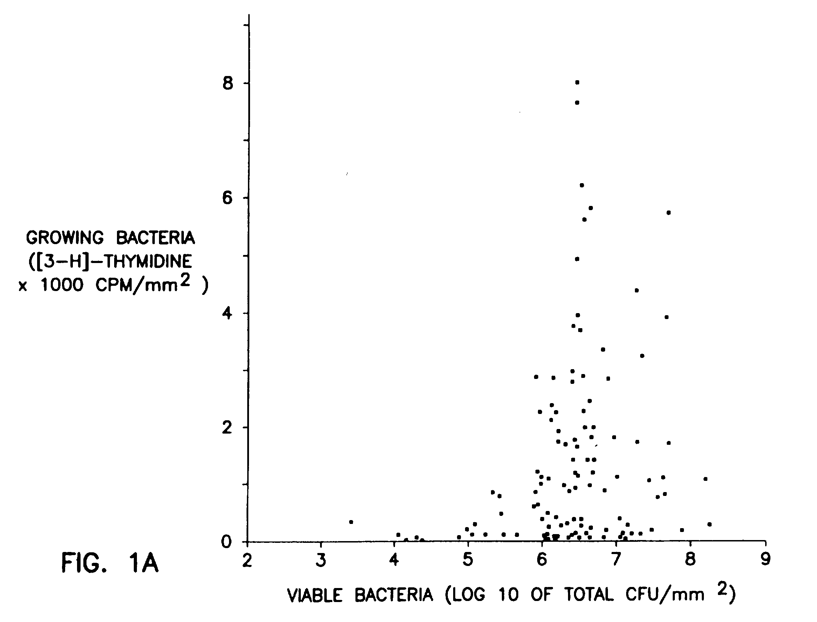

The incorporation of a labeled nucleotide, [methyl-³H]-thymidine, into viable bacteria is illustrated in Fig.1A. In this figure, the number of actively growing bacteria increased greatly as the viable cell density of 2,500,000 - 3,200,000 [10^{6.4-6.5}] was approached. At densities less than 630,000 [10^{5.8}] or greater than 6,300,000 [10^{6.8}] there was a low level of actively growing bacteria. Example 1 contains a description of the processes and techniques used to generate the data shown in Figure 1A.

In another method of the invention, bacterial growth on the surface can be controlled by maintaining a population of bacteria below a density associated with stimulation or enhancement of the proportion of actively growing cells to viable cells. Various surface treatments can either enhance or inhibit adherence and/or growth of bacteria on the surface. Inhibition of adherence and/or growth of bacteria to surfaces can be obtained by limiting the contact with adherence promoting agents such as polypeptides, glycoproteins and carbohydrates. Alternatively, growth and/or adherence of bacteria to surfaces can be inhibited by coating the surface with a hydrophobic substance, preferably a substance that creates a surface having a mean dynamic contact advancing angle in the range of about 60-130° and a mean dynamic contact receding angle greater than about 20°. Suitable examples of such compounds include commercially available products such as SILUX restorative (3M, St. Paul, MN) and VISIO-DISPERS composite (ESPE-Premier, Norristown, PA) and agents disclosed in U.S. Patent No. 5,078,988 issued January 7, 1992.

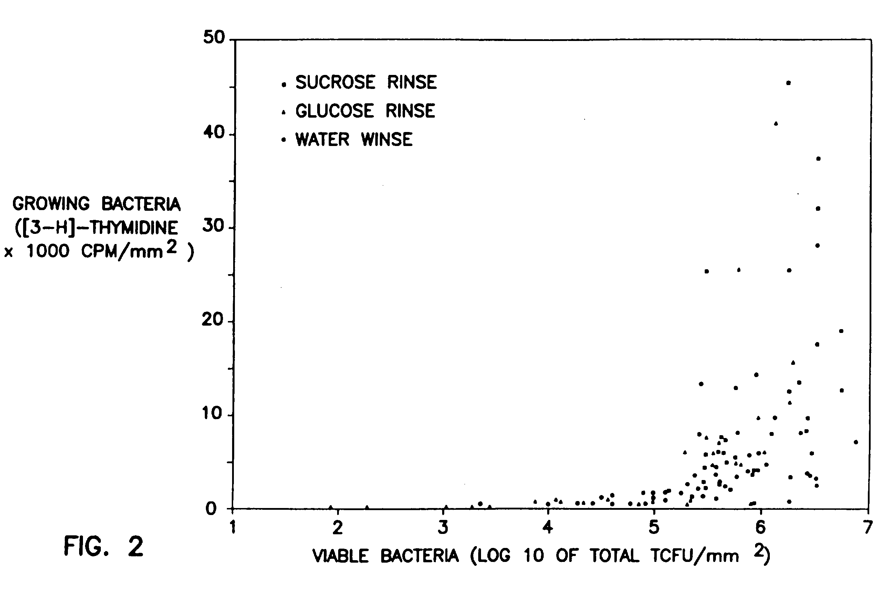

For example, Figure 2 graphically illustrates the incorporation of [methyl-³H]-thymidine into actively growing bacteria taken from implanted bovine enamel surfaces. These implants were exposed to water, glucose or sucrose rinsing in vivo. Sucrose is known to be an adherence promoting agent. As the population density of 2,500,000 - 3,200,000 [10^{6.4-6.5}] is approached, the number of actively growing bacteria dramatically increased. As is described in Example 2, this data indicate that the increase in the rapid growth of bacteria is dependent on density and independent of external factors such as sucrose, glucose or water. Adherence promoting agents can accelerate the time that a population density is reached but do not significantly affect the density at which an increase in rapid growth is seen.

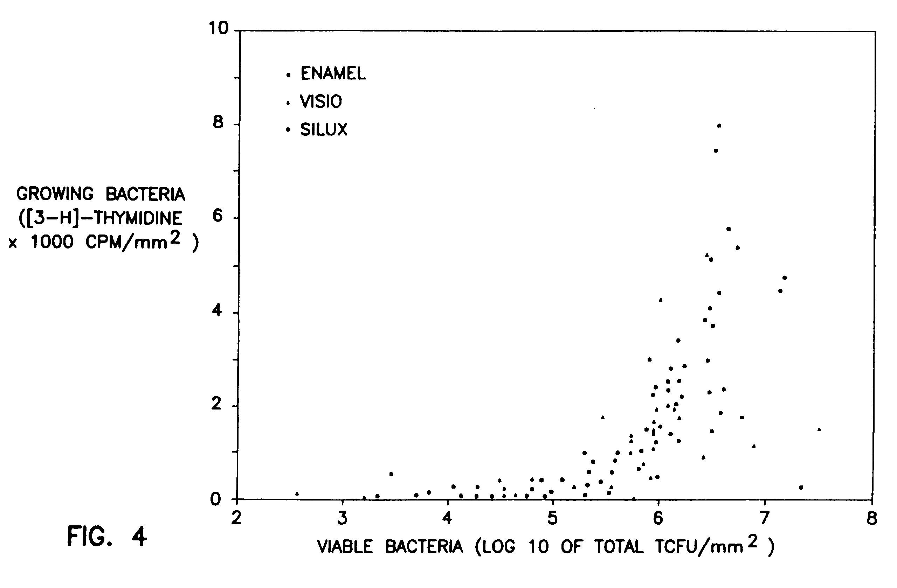

In another example, Figure 4 graphically illustrates the incorporation of [methyl-³H]-thymidine into actively growing bacteria taken from enamel, VISIO-DISPERS or SILUX restorative materials concurrently placed in the mouth. VISIO-DISPERS or SILUX are of materials having hydrophobic surfaces. Again, as the population density of 2,500,000 - 3,200,000 [10^{6.4-6.5}] is approached, the number of actively growing bacteria dramatically increased. As described in Example 4, these data indicate that the increase in rapid growth of bacteria is independent of surface, and dependent on viable cell density. However, hydrophobic surfaces can inhibit adherence and/or growth of bacteria by inhibiting the number of bacteria adhering to the surface and, therefore, the population density may remain below the level associated with density dependent active growth.

In the various embodiments of this invention, the population density of pathogenic or opportunistic bacteria on a surface is treated or controlled to maintain the population density of the bacteria below the density where density growth is observed. Density dependent growth for a bacteria having a size of about 1-2 microns is a density approaching the range of about 400,000 [10^{5.6}] bacteria/mm² to about 6,300,000 [10^{6.8}] bacteria/mm² and, preferably, a density of about 2,500,000 - 3,200,000 [10^{6.4-6.5}] bacteria/mm². While not intending to be limited by theory, it is presently believed that as a bacterial population approaches a population density associated with density dependent growth, there is a communication between the individual bacteria which initiates the onset of active growth in the population. When the bacterial population density is maintained below the density associated with density dependent growth, the prerequisite communication is not sufficient to effect concerted growth of bacteria. Thus, until a density associated with density dependent growth is approached, the detrimental effects of pathogenic or opportunistic bacteria due to growth or metabolic agents are insignificant or maintained at or limited to acceptable levels.

### Microbial Replication Factor (SMR) Compositions

The present invention includes a microbial replication factor designated start microbial replication (SMR), processes for obtaining SMR factor and methods and processes for controlling active bacterial growth using SMR factor or derivatives thereof. SMR factor initiates or starts the processes of active growth or replication of bacteria. Importantly, SMR factor initiates or starts the processes of active growth of bacteria even though the population density of the bacteria is below a density associated with a stimulation or enhancement of actively growing cells in vivo.

When activity of the SMR factor is measured on an bound bacterial cell population, an increase in the proportion of growing cells to viable cells is seen compared with the proportion of growing cells to viable cells in control untreated populations of bacteria. Preferably, the proportion of growing cells to viable cells is a ratio of about 5 X 10⁻⁴:1 to about 20 X 10⁻²:1. The proportion of growing cells to viable cells can be determined as described previously. SMR factor activity is preferably assayed by the methods described in Examples 7 and 11.

SMR factor may be isolated in the bacterial cellfree supernatant of actively growing bacteria. Preferably, SMR factor may be identified in the extracellular matrix surrounding sanguis streptococci. A particularly preferred bacteria is S. gordonii strain Challis ATCC deposit No. 35105. The SMR factor can also be produced by other streptococcal strains including S. gordonii strain 12 and S. gordonii Blackburn as well as by E. coli strain 149, ATTC Deposit No. 55535. E. coli strain 149 been deposited with the ATCC, Rockville, MD on January 7, 1994 and given Accession No. 55535.

SMR factor can be isolated from cells as follows. Sanguis streptococci or E. coli cells are incubated in a buffered media. Both suspension and agar cultures of bacteria are useful in the preparation of SMR factor. As the cells are cultured, extracellular material surrounding the bacteria is collected. When the extracellular material is contacted with bacteria in an in vitro assay, the SMR factor in the material can be determined by the measuring an increase in cellular constituents associated with growth per cell per unit time. In addition, SMR factor activity has also been identified in saliva extracts taken from volunteers having a natural bacterial population present in their oral cavities.

The SMR factor can be isolated from cultures of gram positive and gram negative cells. The isolated SMR factor has a molecular weight less than 3000 as determined by size exclusion membrane filtration. The isolated SMR factor whether derived from gram positive or gram negative cultures can stimulate microbial growth of gram positive or gram negative cultures.

Activity of SMR factor can be assayed by several methods. In one method, a population of bacteria is contacted with a microbial replication factor, preferably for a time sufficient to initiate microbial growth processes. The stimulation of microbial growth is determined by comparing microbial growth in the treated population to microbial growth in an untreated population of bacteria. This method can also be used to determine the effective amount of a SMR factor to stimulate an increase in the proportion of actively growing cells to viable cells in the population, preferably to about 5 X 10⁻⁴:1 to about 20 X 10⁻²:1.

In an alternative method, a bacterial strain having a bacterial reporter gene under the control of a promoter whose expression is coordinately regulated with cell growth as described herein is used to assay the SMR factor. A bacterial strain such as S. gordonii MSP812, ATCC Deposit No. 69528 is contacted with the SMR factor for a time sufficient to initiate microbial growth processes. An increase in the proportion of actively growing cells can be measured by incorporating [methyl-³H]-thymidine into DNA. Alternatively, the increase in actively growing cells can be measured by detecting an increase in the amount of the bacterial reporter gene product per cell per unit time after exposure to the SMR factor compared with the amount of bacteria reporter gene product per cell per unit time in control or untreated cells.

### Methods of Using SMR Factors

SMR factors can be used in methods to inhibit or enhance the growth of bacteria. Agents that inhibit or enhance the activity of the SMR factors can also be identified. Agents that inhibit SMR may be novel therapeutic antimicrobial compounds which can be used to treat undesirable bacterial infections.

In many cases, SMR factors can be used to stimulate or enhance the growth of bacteria. Many types of bacteria are used in industrial processes. Addition of SMR factor may increase the yield of bacteria or the efficiency of growing the bacteria. In addition, SMR factor could be added to growth media, especially those used in diagnosis of bacteria. The stimulation of microbial growth in the media may allow for an earlier diagnosis of infection. There are many other examples where stimulation or enhancement of microbial growth may be beneficial.

The invention provides a method for stimulating microbial growth. The steps of the method involve contacting a microbial population with an amount of an SMR factor effective to enhance the growth of the cells, i.e., increase the proportion of actively growing cells to viable cells in the population. The effective amount of SMR factor for a particular bacteria can be determined by conducting a standard dose response test using a method such as described in Example 7. An effective amount of SMR factor is that amount that may provide a ratio of growing cells to viable cells in a population as high as 20 x 10⁻²:1.

The SMR factor can be contacted with bound bacteria or bacteria grown in liquid culture. The SMR factor can be left in the culture medium and may be added from time to time as media components become depleted or if the bacteria are grown in a continuous culture system.

A method is also provided to identify agents that inhibit or enhance SMR factor. The steps of the method involve contacting a population of bacteria with a SMR factor and an agent suspected of inhibiting or enhancing the activity of SMR factor to form a treated population. The population is incubated for a time sufficient to detect initiation of microbial growth. The proportion of actively growing cells to viable cells in the treated population is compared to the proportion in a population treated with SMR factor alone. An enhancement or increase in the proportion may indicate that the agent is another microbial growth factor. An inhibition of the proportion of actively growing cells may indicate that the agent may be useful as an antimicrobial agent.

### Genetic Cassettes

The invention also provides a genetic cassette comprising a first DNA sequence coding for a bacterial reporter gene linked to the 3' end of a second DNA sequence. The second DNA sequence is hybridizing to a gene or portion thereof whose expression is coordinately regulated with cell growth. The gene is located in the bacterial chromosome. The second DNA sequence in the cassette functions to provide for recombination and integration of the genetic cassette or a portion thereof into the bacterial chromosome. Integration of the cassette into the bacterial chromosome provides for expression of the bacterial reporter gene product under the control of a promoter whose expression is coordinately regulated with cell growth.

A bacterial reporter gene product is a polypeptide having a structural or functional characteristic which can be measured. In this invention, the bacterial reporter gene product functions to identify cells which are actively growing. For example, if a bacterial reporter gene product is an enzyme, the expression of the bacterial reporter gene product can be detected by measuring the enzyme activity. Alternatively, the bacterial reporter gene product can be measured by a change in the characteristics of the cell such as the color of the colony. The bacterial reporter gene product is preferably not present in the host cell into which the bacterial reporter gene becomes integrated. In addition, the bacterial reporter gene product is preferably not toxic to the bacterial host cell.

Suitable examples of bacterial reporter gene products are enzymes such as amylase, β-galactosidase, chloramphenicol-acetyl transferase, and luciferase. A preferred bacterial reporter gene product for S. gordonii Challis ATCC No. 35105 strains is amylase as S. gordonii Challis ATCC No. 35105 does not have endogenous amylase activity.

DNA sequences coding for bacterial reporter genes can be obtained using standard methods as described in Ausubel et al., Current Protocols in Molecular Biology, Greene Publishing Associates and Wiley Interscience (1987). The DNA sequences coding for a specific reporter genes are commercially available in plasmids or can be obtained by reference to published methods. For example, a DNA sequence coding for a bacterial amylase was obtained in a plasmid designated pRQ200 from Dr. Ron Yasbin, University of Maryland, Baltimore, Maryland, constructed as described in Lane et al., Gene, 100:225-229 (1991). The DNA sequences coding for a bacterial reporter gene product are preferably not present in the genome of the bacterial host cell and therefore are preferably heterologous DNA sequences obtained from other species or genera of bacteria.

DNA sequences coding for a bacterial reporter gene can be amplified using appropriately designed primers in PCR or by subcloning into a plasmid that can be amplified in a bacterial cell. Once amplified, a DNA sequence coding for a bacterial reporter gene can be combined with a second DNA sequence hydrizing to a gene or portion thereof whose expression is coordinately regulated with cell growth to form an expression cassette. The first DNA sequence is combined with the second DNA sequence so that integration of the first DNA sequence into the bacterial chromosome is in the proper orientation for expression. The 5' end of the first DNA sequence is combined with the 3' end of the second DNA sequence. The first and second DNA sequences can be combined by methods known in the art such as described in Example 10.

A genetic cassette of the invention also includes a second DNA sequence linked to the first DNA sequence. The second DNA sequence hybridizes to a gene or portion thereof whose expression is coordinately regulated with cell growth and is located in the bacterial chromosome. The function of the second DNA sequence is to provide for recombination and integration of the genetic cassette or a portion thereof into the bacterial chromosome. Bacterial cells preferably have the genetic cassette integrated into the chromosome at a location that is under control of a promoter whose expression is coordinately regulated with cell growth. The second DNA sequence can target the integration of the genetic cassette or portion thereof to a specific location in the chromosome by selecting a second DNA sequence that hybridizes to a gene or portion thereof whose expression is known to be coordinately regulated with cell growth. The 5' end of the first DNA sequence encoding a bacterial reporter gene is linked to the 3' end of the second DNA sequence.

The second DNA sequence hybridizes to a gene or portion thereof whose expression is known to be coordinately regulated with cell growth or a random DNA sequence in the bacterial chromosome of a selected bacterial strain. Preferred bacterial strains include streptococcal strains such as S. gordonii Challis ATCC Deposit No. 35105. Genes whose expression is known to be coordinately regulated with a selected bacterial strain are known to those of skill in the art such as the ribosomal RNA genes, and genes encoding ribosomal proteins. The genes are preferably also present in multiple copies in the bacterial chromosome so that integration of a genetic cassette or portion thereof into the gene will not disrupt an essential function of the bacteria and/or result in the death of the transformed cells. The DNA sequences of genes or portions of genes whose expression is coordinately regulated with cell growth or DNA sequences for random segments of the bacterial chromosome in a selected bacterial strain can be determined by reference to scientific publications or by searching genetic sequence databases such as Genbank at National Institutes of Health, Bethesda, Maryland, or RNA data base at University of Illinois/Champaign/Urbana, Champaign, Illinois.

The second DNA sequence has sufficient sequence identity to the complementary strand of portion of a gene or random segment of the bacterial chromosome to provide for recombination and integration of all or a portion of the genetic cassette into the gene or the random location in the bacterial chromosome. Sufficient DNA sequence similarity for recombination and integration to occur is about 100 to 5000 nucleotides of contiguous DNA sequence with 80-100% identity with the complementary strand of the portion of the selected gene or the random DNA sequence of the bacterial chromosome. Preferably, the second DNA sequence has sufficient similarity to the complementary strand of a gene or portion thereof to provide a high enough frequency of integration to readily detect integrants. Such frequencies are preferably about 10⁻⁶ to 10⁻⁸.

A preferred portion of a gene is an internal fragment of the streptococcal 16S rRNA gene described in Example 10. The second DNA sequence also preferably contains additional DNA sequences of about 4 to 8 nucleotides long on both ends to provide for restriction endonuclease cleavage sites. The sequence of restriction endonuclease cleavage sites are known to those of skill in the art. The sequence at the ends can be the same restriction endonuclease recognition sequence or different recognition sequences. These sequences allow one of skill in the art to select appropriate restriction sites to allow for cloning into a selected location in a vector to insure that the first DNA sequence is linked to the 3' end of the second DNA sequence.

Once the second DNA sequence is selected, it can be prepared in any number of ways. For example, primers complementary to portions of the second DNA sequence can be prepared as described in Example 10. The primers can then be used to amplify a chromosomal DNA sequence coding for a gene or portion thereof whose expression is coordinately regulated with the rate of cell growth using polymerase chain reaction (PCR). Alternatively, portions of the selected gene can be subcloned by standard methods including using restriction endonuclease digestion and ligation into a vector that can be amplified. The selected random DNA sequences can be synthesized by automated DNA synthesis, amplified using PCR or by subcloning.

A DNA probe or primer useful in PCR is about 15 to 50 nucleotides long and has a sequence complementary to the gene or portion thereof or the random segment in the bacterial chromosome. The primer or probe can be prepared by automated DNA synthesis. Examples of such primers are the primers that are complementary to the DNA sequence coding for a 16S ribosomal RNA in streptococcal strains as shown in Example 10. Other primers or probes can be designed by examining the DNA sequence of a portion of a gene or the random DNA sequence. Preferably, primers and/or probes are designed to be complementary to a portion of the gene whose DNA sequence is conserved in other strains of bacteria. For example, the primers that are complementary to a portion of a ribosomal RNA sequence were selected by comparing the sequence of several 16S rRNA genes and identifying conserved regions of DNA sequence. A conserved region of DNA sequence preferably shares about 90 to 100% DNA sequence identity.

A genetic cassette further comprises a selectable marker gene. The selectable marker gene is linked to the first and second DNA sequences, usually by subcloning of those sequences into a vector carrying the selectable marker gene. Selectable marker genes are preferably antibiotic resistance genes and are available in many commercially available vectors.

Once a second DNA sequence is selected and obtained, the second DNA sequence is combined with the first DNA sequence so that the 5' end of the first DNA sequence is linked to the 3' end of the second DNA sequence. This combination of DNA sequences can be accomplished by standard methods of subcloning into a vector such as a plasmid. The second DNA sequence can be prepared with selected restriction endonuclease sites at either end to provide for ease of cloning into a plasmid. The first DNA sequence coding for a reporter gene can already be present in the plasmid or can be subcloned downstream from the second DNA sequence.

The vectors which are suitable for the invention are vectors that can be used to introduce the genetic cassette into bacterial cells and to provide recombination and integration of the genetic cassette or a portion thereof into the bacterial chromosome. Suitable vectors include plasmids and viruses. Plasmids are selected so that they do not replicate in the selected bacterial host cell so that integration of the genetic cassette is favored. Many vectors are commercially available or can be constructed by published methods. The vector preferably includes a selectable marker gene. A preferred plasmid is the pRQ200 plasmid described in Example 10.

### Bacterial Strains Having a Genetic Cassette Stably Integrated

Once a genetic cassette is prepared according to the invention, it is introduced into a host cell. Preferably, the host cell is a streptococcal strain. However, other bacteria may also serve as a suitable host cell. Preferably, the genetic cassette is introduced into the cell in a vector. The vector selected is preferably one that does not replicate in the bacterial host cell so that integration of the genetic cassette is favored.

The genetic cassette can be introduced into a bacterial host cell using standard methods such as calcium phosphate precipitation and electroporation. If the bacterial cells are competent for transformation such as S. gordonii Challis ATCC Deposit No. 35105 then the DNA can be mixed directly with the cells. After the genetic cassette is introduced into the bacterial host cells, transformed cells are selected.

Transformed cells can initially be selected for the presence of a selectable marker gene present on the transforming vector. If the vector does not replicate in the host cell, the transformed cells carrying the selectable marker gene have all or a portion of the vector integrated into the chromosome. Recombination mechanism in many bacteria are known to those of skill in the art. Recombination can occur as single or double crossover events so that the amount of the vector integrated into the chromosome can vary. At a minimum, transformed cells have an integrated selectable marker gene and a functional bacterial reporter gene. Transformed cells also preferably also contain a selectable marker gene.

Transformed cells can be screened for the expression of the bacterial reporter gene product. The assays for the bacterial reporter gene products are known to those of skill in the art. Transformed cells can further be selected for coordinately regulated expression of the bacterial reporter gene product with cell growth.

The expression of a reporter gene product in actively transformed growing cells can be measured in the presence or absence of microbial growth factors using standard methods. Coordinately regulated expression of a reporter gene product can be determined by measuring an increase in reporter gene product per cell per unit time in the presence vs. absence of microbial growth factors.

Specific examples of selection methods and bacterial strains are described in Examples 10 and 11. Of 20 transformants, 2 were selected for further characterization. One strain that has been shown to produce a microbial replication factor is S. gordonii Challis MSP812. S. gordonii Challis MSP812 has been deposited with the American Type Culture Collection, Rockville, MD on January 6, 1994 and given Accession No. 69528. Another strain which produced more amylase per unit time in actively growing cells is S. gordonii Challis MSP818. S. gordonii Challis B2B MSP818 has been deposited with the American Type Culture Collection, Rockville, MD on January 6, 1994 and given Accession No. 69529.

### Methods of Measuring Microbial Growth

The bacterial strains having a stably integrated expression cassette under control of a promoter whose expression is coordinately regulated with cell growth can be used to measure microbial growth. Microbial growth can be stimulated by inoculating the bacterial cells in a medium containing all of the requirements for growth of this particular bacterial strain. Media and conditions for growth are known to those of skill in the art for different bacterial strains. Growth in the population can be monitored by determining the amount of the reporter gene produced per unit time.

Alternatively, the bacterial strains can be used to measure the activity of a start microbial replication factor as shown in Example 11. The bacterial strains are contacted with a start microbial replication factor and incubated for a time sufficient for the growth factor to stimulate an increase in the proportion of actively growing cells to viable cells in the population. An increase in bacterial reporter gene product per cell per unit time can be measured in a population of bacteria in response to a start microbial replication factor compared with an untreated population of bacteria.

### Examples

The following examples are provided to further illustrate specific embodiments of this invention. These examples are intended to further describe the present invention but should not be used to limit the scope of the appended claims.

### EXAMPLE 1 - Determination of Actively Growing Populations On Enamel Inserts

Bacterial colonization of teeth in the mouth of 20 adults was studied using an in vivo model. Plaque was examined in healthy adult subjects who continued with their normal diet. (Selection criteria included the absence of systemic disease, pregnancy or extensive restorations. The subjects were instructed to maintain a normal diet and oral hygiene; excluding brushing the actual bonded test materials (see below) or rinsing with any commercial mouth rinse.)

Bovine enamel pieces having a measured area of about ten mm² were directly fixed to six contralateral upper premolars and first molars (tooth numbers 1·4, 1·5, 1·6, 2·4, 2·5, and 2·6) within custom enclosures, so that the enamel pieces could be easily inserted and removed using established methods. Enamel piece construction and enclosure in vivo are described in detail below. Following placement on 6 teeth per subject, the pieces of enamel were left for varying time periods to accumulate dental plaque. The enamel pieces covered with bacterial plaque were removed from the mouth after 2, 4, 8, or 24 hours and examined for viable microbial composition, total viable bacteria, and the proportion of the population actively growing using methods described in detail below.

These data generated information on the number of viable cells, the composition of microbial species of those viable cells, a measure of the portion of the population actively growing. Colonization of implanted surfaces, or biofilm formation, is defined as specific adherence plus cell division. Mechanisms suggested to be significant to the eventual plaque bacterial biomass include (1) adherence of indigenous bacteria to specific receptors on enamel pellicular surfaces; (2) coaggregation or adherence between bacteria already adherent to enamel (preformed plaque) and other indigenous bacteria (interbacterial adherence or coaggregation); and (3) growth or cell division of naturally adherent bacteria. Only the first mechanism has been directly studied in vivo.

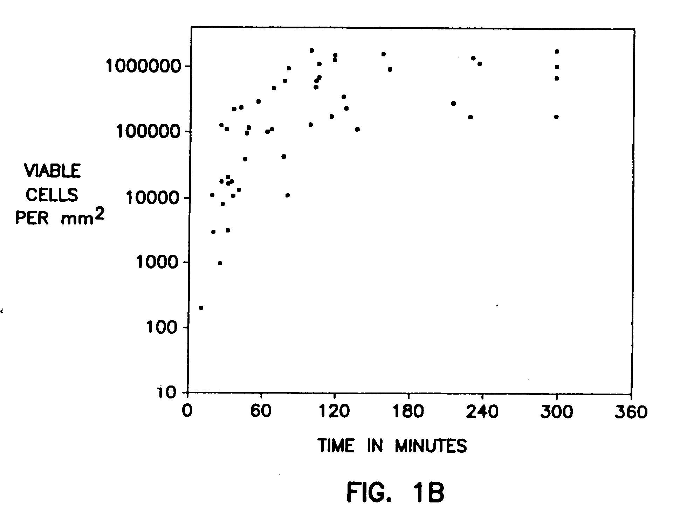

Based on data from these studies, natural adherence can be evaluated in two ways. First, the mean viable counts per mm² has been reported. Liljemark et al., Oral Micro. & Immun., 8:5-15 (1993). After 4 hours in the mouth, about 1-2,000,000 viable cells/mm² are found. The second method of evaluating the contribution of adherence to plaque biomass is exhibited in Figure 1B. By examining each tooth location in 18 subjects, saturation of pellicle binding sites can be observed. This graphical representation shows that the pellicle sites are saturated between 200,000 and 630,000 [10^{5.3} to 10^{5.8}] viable cells/mm². This corresponds to in vitro measurements of available salivary binding sites for the oral streptococci.

Based on data from similar studies, the in vivo interbacterial adherence rate was measured by inserting enamel implants covered with a continuous surface of streptococci (a strain reported to display most of the recognized coaggregation surface adhesins which cause in vitro inter-species bacterial clumping in buffer). Skopek et al., Oral Micro. and Immun., 8:16-23 (1993). Similarly, the in vivo adherence rate to implants was measured. Liljemark et al., cited supra.

Given that about 33 million bacteria per mm² form the plaque biomass by 24 hours, it is apparent that cell division is a major contributor to plaque biomass in the first day of plaque formation. Adherence at saturation to specific sites on pellicle accounts for only 1.5% of the 24-hour plaque biomass. Adherence to enamel or due to interbacterial adherence accounts for about 10-20% of the plaque biomass by 24 hours in the mouth.

These measurements of the accumulation of indigenous bacteria do not measure whether plaque bacteria were actually dividing. Growth of bacteria can be studied and is apparent when the relationship between the actively growing proportion of the plaque population is graphically illustrated based on viable cell density of individual sites (see Figure 1A). At a density approaching 2,500,000 - 3,200,000 [10^{6.4.6.5}] bacteria/mm², independent of the length of time it took to reach this density, there was a significant increase in the number of actively growing cells. This large increase in the number of actively growing bacterial cells at this population density indicates that adherent bacteria do not divide or grow slowly, but instead enter a period of rapid growth.

The method to construct and enclose enamel implants in vivo were as follows. Enamel pieces from extracted bovine teeth were cut into squares about 3 x 3 x 1 mm with 45° divergent walls. The pieces were measured using a SHERE-TUMICA digital caliper (Shere-Tumica, St. James, MN) and stored in distilled water at 4°C until used. Custom made enclosures were fabricated on a stone model of an alginate impression of the maxillary arch of each subject. The enamel pieces were pressed into a ball shaped piece of VISIO-FIL composite (ESPE-Premier, Norristown, PA) which was applied to the facial surface of the maxillary premolar and molar teeth. The composite was formed to be smooth and continuous with the enamel of the tooth and the enamel piece seated into the composite so that only its surface was exposed. The enamel pieces were then placed into the enclosures which were removable utilizing the tip of a sharp explorer via a minute notch made during fabrication of the enclosures. The enamel pieces fitted tightly into their respective enclosures preventing plaque formation anywhere except on the exposed surface. The custom enclosures were then applied to the respective teeth using a CONCISE orthodontics bonding system (3M, St. Paul, MN).

The methods to measure microbial composition and total viable cells were as follows. The enamel pieces were placed in 1.0 ml prereduced anaerobically sterilized (PRAS) diluent (L. V. Holdman and W. E. C. Moore, Eds. Anaerobic Laboratory Manual, Virginia Polytechnic Institute and State University, Blacksburg, VA, 124-127, 1973)) in an anaerobic chamber (Coy Anaerobic Chamber, Ann Arbor, MI) and gently sonified for 20 seconds (Kontes Cell Disruptor; Kontes, Vineland, NJ). An aliquot of the plaque sample was serially diluted and plated (Spiral Systems, Inc., Cincinnati, OH).

Cultivation of the various bacterial species was accomplished on a variety of elective and selective media. Total cultivable anaerobic microbial flora was determined using supplemented blood agar (SBA) medium containing Trypticase soy agar (BBL Microbiology Systems, Cockeysville, MD), 5% defibrinated sheep blood, 0.00005% menadione, 0.0005% hemin and 0.001% NAD (Sigma Chemical Company, St. Louis, MO) and incubated anaerobically at 37°C for seven days. Dark-pigmented anaerobic rods, including the species Porphyromonas gingivalis, Prevotella intermedia and Prevotella melaninogenicus, were enumerated on SBA media. Fusobacterium spp. were identified as round, entire, smooth iridescent colonies on SBA media. Streptococcus spp. were enumerated on Mitis salivarius (MS) agar (Difco Labs, Detroit, MI) with 0.1% potassium tellurite (Difco Labs, Detroit, MI) added. These plates were incubated anaerobically in an atmosphere of 10% hydrogen - 10% carbon dioxide - 80% nitrogen at 37°C for 2 days and then for 24 hours aerobically at room temperature. Adherent hard colonies, which would include the species Streptococcus sanguis, Streptococcus gordonii, and Streptococcus oralis, and referred to as sanguis streptococci, were identified visually by colonial morphology using a stereomicroscope and indirect light according to known methods. Mitis streptococci were round, smooth and creamy and included the species Streptococcus mitis and non-adherent S. oralis colonies.

Mutans streptococci were enumerated on MS agar and on MS-bacitracin media using well known methods. The colony counts of mutans streptococci shown were the ones highest on either of the two media used. Veillonella spp. were enumerated on the modified medium of Rogosa-SL agar (Difco Labs, Detroit, MI) and identified by colonial morphology and gram stained smears. Haemophilus spp. were enumerated on modified chocolate (MC) agar, following incubation in 10% carbon dioxide at 37°C for 3 days according to known methods (Liljemark et al, Infect Immun. 46: 778, 1984). Actinomyces spp. were enumerated on CFAT agar (Zylber and Jordan, J. Clin. Microbiology, 15:253-259, 1982). Representative colony types from each subject were first tested for reaction to catalase. Subsequently these strains were reacted with antisera kindly provided by Dr. George Bowden (University of Winnipeg, Winnipeg, Canada). Utilizing this system Actinomyces were speciated into Actinomyces viscous (resembling Actinomyces naeslundii II), Actinomyces naeslundii I and Actinomyces odontolyticus. Total cultivable anaerobic microbial flora (viable cells) and total colony-forming units of species were calculated per mm² surface.

The actual number of actively growing bacteria on the pellicular surface was measured using the incorporation of radiolabelled [methyl-³H]-thymidine and [C¹⁴]-adenine. To this end, plaque samples were incubated for 30 minutes, and the radiolabelled target molecules DNA and/or RNA were extracted from the plaque samples, and counted. As DNA synthesis occurs throughout the life cycle of a bacterial population, these nucleotides will label all actively growing cells. Labelling DNA is also useful as it does not turn over and because synthesis does not occur in non-growing cells. By calculating the amount of incorporation of [methyl-³H]-thymidine and dividing by the total number of viable cells, cpm/bacteria can be calculated and this ratio will reflect a change in the number of actively growing cells as a proportion of the total viable cells. Incorporation of radiolabelled nucleotide into TCA insoluble materials ensures measurement of actual DNA synthesis rather than metabolism of radiolabelled nucleotides. The short period (compared to minimum cell division time), use of excess [methyl-³H]-thymidine and [C¹⁴]-adenine and the repeated sampling over time in a manner not disruptive to plaque formation was found to be a novel method to determine growing bacterial populations.

The measurement of actively growing population are as follows. The incorporation of [methyl-³H]-thymidine and [C¹⁴]-adenine into trichloroacetic acid (TCA) insoluble material of the timed bacteria plaques was done by mixing an aliquot of sonified plaque (0.3 ml) with 0.1 ml of a mixture of [methyl-³H]-thymidine (45,000 mCi/mmol, Research Products International Corp., Mt. Prospect, IL) and [C¹⁴]-adenine (270 mCi/mmol, Amersham Research Products, Arlington Heights, IL). The final concentration of nucleosides when mixed with the plaque samples was 150 mCi/ml (100-200 nmoles) [methyl-³H]-thymidine and 65 mCi/ml (200-250 nmoles [C¹⁴]-adenine). The plaque and radiolabelled nucleosides were mixed and incubated within the anaerobic chamber for 30 minutes. Radiolabelled plaque samples were removed from the chamber, incubation terminated by the addition of 2.0 ml of cold (4°C) 10% (wt/vol) TCA and extracted for an additional 30 minutes at 4°C. The precipitate was collected on Gelman GN-6 cellulosic filters (Gelman Sciences, Ann Arbor, MI) and rinsed 3 times with 5.0 ml of cold 10% TCA. The filters were placed in vials, dried and scintillation cocktail added for counting. Radiolabel incorporation was expressed as [³H] or [C¹⁴] counts per minute (CPM) per mm² of enamel surface or per bacteria (as determined by total CFU). Radiolabel was in excess. For example, if an Escherichia coli cell contains 9 x 10⁻¹⁵ grams of DNA per cell and 25% of the DNA is thymidic residues, then over one million times the genomic equivalent of thymidine of 10^{6.0} bacteria was added to each reaction mixture. A million excess genomic equivalents of adenine were added. The pulse labeling avoided the difficulties of determining intracellular specific activity and isotopic equilibration because each sample was pulsed for the same short time period.

Incorporation of [methyl-H³]-thymidine into bacterial cells at the density where saturation of pellicular binding sites occurred (about 200,000 - 630,000 [10^{5.3} - 10^{5.8}]) was about 3.7 x 10⁻⁴ cpm/bacteria. The total cpm of [methyl-H³]-thymidine per viable cell at this time of active growth was on the average of about 20 x 10⁻⁴ cpm/bacteria. When bacterial cells in the plaque samples are given a steady source of energy for growth, such as sucrose rinse, the cpm/bacteria was 160 x 10⁻⁴ at a density approaching about 2,500,000 to 3,200,000 cells [10^{6.4-6.5}]. See Example 2.

**TABLE 1**

| **Comparison of** ^{**3**}**H-thymidine Incorporation as a Measure of DNA Synthesis in Streptococci In Vivo and In Vitro** | | |
|---|---|---|
| | **Density per mm**^{**2**} | **(**^{**3**}**H-thymidine cpm/ bacteria) x** |
| **10**^{**-4**} | | |
| Overnight culture in Todd Hewitt media | [10⁹ bacteria per ml. liquid] | 2.5 |
| | | |
| Predominantly streptococci adhering to enamel at saturation^{a} | 200,000 - 630,000 | 3.7 |
| | | |
| Bacteria in dental plaque^{b} on enamel, SILUX or Visio-Dispers | 2,500,000 - 3,900,000 | 20 (160 with sucrose rinse) |
| | | |
| Bacteria immobilized to maleic anhydride plates and media^{c} | 625,000 | 5.4 |
| | | |
| Bacteria immobilized to maleic anhydride plates and START^{c} | 625,0000 | 80 |

| | | |
|---|---|---|
| ^{a} in vivo data, mean 80% of 16 sites | | |
| ^{b} in vivo data, mean 80% of 22 sites | | |
| ^{c} in vitro assay, mean 80% of 35 experiments | | |

### EXAMPLE 2 - Examining the Effects of Rinsing with Sucrose Glucose or Water on Cell Growth

Eight healthy adults, 4 males and 4 females, participated in the study. Bovine enamel pieces were mounted on the maxillary first molars and first and second premolars according to the methods described in Example 1.

Experiments were conducted for 6 hours on all 8 subjects with 6 enamel pieces per subject. Each subject rinsed her mouth with 10 ml of either water, 10% glucose (sucrose free glucose w/v) or 10% sucrose (w/v) (Sigma, St. Louis, Missouri ) every 15 minutes, for 1 minute, for the 6-hour duration of the experiment. On day one subjects rinsed with glucose, on day two with water, and on day three with sucrose. After 2 hours in vivo, 2 of the enamel pieces were randomly removed from their enclosures. After 4 hours, 2 more enamel pieces were removed as above and, after 6 hours, the final 2 pieces were removed and processed. Immediately after removal from the mouth the plaque samples were processed for viable cell counts composition described in Example 1.

Sucrose is a known adherence promoting and bacterial growth promoting substance. It was expected that in the presence of excess sucrose, that the indigenous bacterial population would immediately show a high level of active growth as they adhered to enamel implants. It was unexpected to find that, similar to indigenous plaques described in Example 1 under conditions of normal diet, that the viable adherent bacteria increased their level of active growth as they approached the density of 2,500,000 - 3,200,000 [l0^{6.4-6.5}]. It is not unexpected that the level of active growth exceeded that of enamel implants without rinsing. What is unexpected is that this increase occurred as the viable cell density approached 2,500,000 - 3,200,000 [10^{6.4-6.5}]. Additionally, it was unexpected that both glucose and water rinsing increased the number of actively growing cells.

### EXAMPLE 3 - Control of Population Density Using an Adherence Suppressing Agent

Bovine enamel chips were mounted on teeth as previously described in Example 1. Solutions of octadecyl sulfate, sodium salt (Aldrich Chemicals Co., Milwaukee, WI) were formed at 60°C from deionized water and weighed proportions of sodium octadecyl sulfate (SOS) sufficient to make 5 X 10⁻³ M. The actual concentration may be somewhat less than 5 X 10⁻³ due to the solubility of SOS in water at room temperature. Only the clear supernatant was used.

Enamel pieces were incubated with the clear supernatant of SOS for 30 minutes and then washed for 5 minutes with water to remove excess solution, leaving only the oriented monomolecular layer of octadecyl sulfate. Enamel pieces with bound SOS are called SOS-enamel.

Whole saliva, stimulated with parafilm, was collected from two persons and clarified by centrifugation at 10,000 x g for 10 minutes. One mL of the clarified and mixed saliva was then incubated with enamel pieces or with SOS-enamel pieces and incubated at room temperature for 45 minutes with gentle shaking. Controls were incubated with phosphate buffer. Gibbons and Etherden, Infect. Immun., 36:52-58 (1982). The saliva-coated enamel pieces (SC-enamel) and saliva-coated SOS-enamel pieces (SC-SOS-enamel) were washed also with phosphate buffer. Gibbons and Etherden, cited supra.

Enamel, SOS-enamel, SC-enamel and SC-SOS-enamel were assessed for hydrophobicity in a Wilhelmy Balance (Cahn Instruments, DCA-322, Cerritos, CA). The piece perimeter was 17.84 mm. Time interval of immersion was one second. Platform speed was 148.39 microns/sec. The dynamic contact angles for enamel and enamel treated with SOS, and SC-SOS-enamel are illustrated in Table 2.

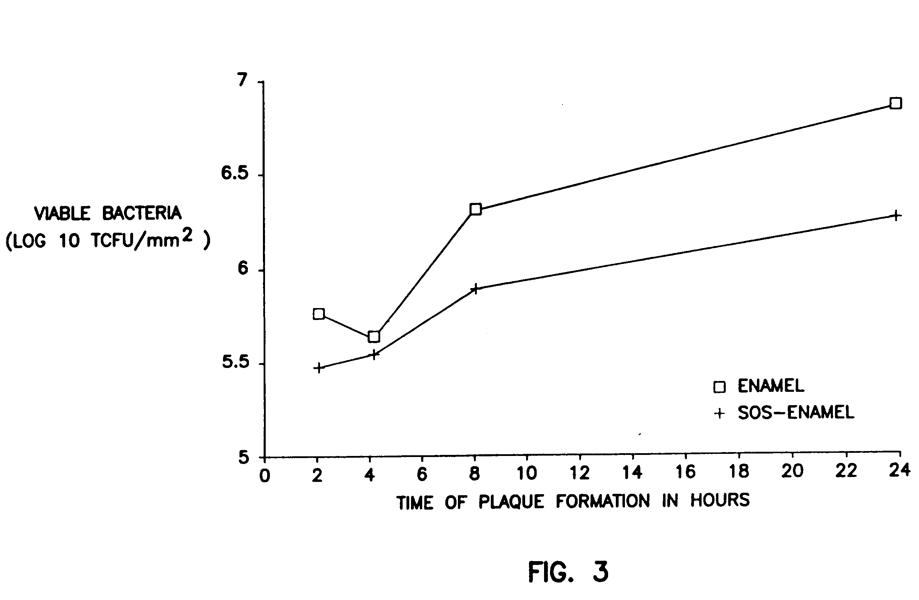

The effect of SOS treated enamel on in vivo plaque formation was compared to enamel in two subjects using a random placement of 3 pieces of enamel and 3 pieces of SOS-enamel in custom enclosures as described in Example 1. Each subject was examined twice for plaque formation after 2, 4, 8, andn 24 hours. Total viable bacteria were enumerated on the enamel and SOS-enamel as described in Example 1. Greatly lower viable bacteria (geometric mean) were found on SOS-enamel versus enamel after 2, 4, 8, and 24 hours, as illustrated in Fig. 3.

**TABLE 2**

| ENAMEL | | | |
|---|---|---|---|
| Angle | CYCLE | | |
| | 1 | 2 | 3 |
| Ad | 40 | 0 | 0 |
| Re | 0 | 0 | 0 |
| | | | |
| Ad | 64 | 35 | 29 |
| Re | 0 | 0 | 0 |

| SOS-ENAMEL | | | |
|---|---|---|---|
| Angle | CYCLE | | |
| | 1 | 2 | 3 |
| Ad | 103 | 71 | 56 |
| Re | 13 | 19 | 22 |
| | | | |
| Ad | 103 | 60 | 60 |
| Re | 28 | 33 | 34 |

| SC-SOS-ENAMEL | | | |
|---|---|---|---|
| Angle | CYCLE | | |
| | 1 | 2 | 3 |
| Ad | 57 | 0 | 0 |
| Re | 0 | 0 | 0 |
| | | | |
| Ad | 68 | 51 | 49 |
| Re | 33 | 34 | 36 |

### EXAMPLE 4 - Control of Population Density Using Hydrophobic Materials

Bacterial colonization of teeth in the mouth of 8 adults was also studied using a similar in vivo model. The 8 adult subjects used in this study were dental students or professional microbiologists. Pieces of bovine enamel, SILUX restorative (3M, St. Paul, MN), VISIO-DISPERS light-cured composite dental restorative (ESPE-Premier, Norristown, PA) (each piece having an area of about 10 mm²) were directly and concurrently fixed to 6 contralateral premolars and first molars, within custom enclosures, and left for varying time periods to accumulate dental plaque bacteria.

Bovine enamel pieces were cut into squares from extracted bovine teeth as described in Example 1 above. The composite resin pieces, VISIO-DISPERS light-cured composite dental restorative (ESPE-Premier, Norristown, PA), and SILUX restorative (3M, St. Paul, MN), were formed in a model made from EXPRESS vinyl polysiloxane registration material (3M, St. Paul, MN) in which a preformed bovine enamel piece was used to form a mold in the EXPRESS polysiloxane material. The composite material was applied to the preformed mold and a mylar strip was pressed over the top to form a smooth and even surface. The composite was then light cured for 40 seconds. The composite resin pieces were removed from the mold, the edges finished and the surface area measured. The outer surface of the pieces were polished with medium and fine SOF-LEX disks (3M, St. Paul, MN) to simulate composite material applied in a clinical setting and to remove the resin rich layer.

Custom made piece enclosures were fabricated as described in Example 1. Cultivation of the various bacterial species was accomplished on a variety of elective and selective media as described in Example 1. Enamel pieces were removed at prescribed intervals and placed in 1.0 ml of PRAS diluent as described in Example 1 and sonified. The incorporation of [methyl-³H]--thymidine and [C¹⁴]-adenine into trichloroacetic acid insoluble material of the timed bacterial plaques was done as described in Example 1.

Fig. 4 illustrates that fewer pieces of VISIO-DISPERS (ESPE-Premier, Norristown, PA) or SILUX (3M, St. Paul, MN) showed a rapid increase of actively growing bacteria because fewer pieces approached the population density associated with stimulated or active bacterial growth. These surfaces were more hydrophobic than enamel when examined using the Wilhelmy balance for relative hydrophobicity and saliva-coated VISIO-DISPERS (ESPE, Norristown, PA.) and saliva-coated SILUX (3M, St. Paul, MN) were also more hydrophobic than saliva-coated enamel. Specifically, the mean (±SE) receding angle measurements of 8 replicates were as follows: Enamel 16.1 (±2.3), SC-Enamel 4.99 (±2.6), VISIO-DISPERS composite 23.5 (±0.64), SC-VISIO-DISPERS composite 14.1 (±2.3), SILUX restorative 29.2 (±2.7), SC-SILUX restorative 14.6 (±0.89). Saliva coating of all surfaces were significantly less hydrophobic than the uncoated parent surface (paired t-test). Additionally SC-SILUX restorative particles were significantly more hydrophobic than SC-Enamel, repeated measures ANOVA, Scheffe f test. It was unexpected to find this hydrophobic "shine through", and that the deposited salivary pellicle was presumably modified to mediate a significant part of its hydrophobicity.

### EXAMPLE 5 - Preparation of SMR Factor-Containing Compositions

Compositions containing SMR factor were isolated from both suspension and agar cultures of S. gordonii strain Challis ATCC deposit No. 35105. Briefly, an inoculating scraping from an SBA plate containing Challis strain (a lawn of cells were cultured overnight on SBA) was added to a Klett flask (300 ml capacity, one plate per 20 ml TH media), and the inoculum (Klett about 25) was cultured in Todd Hewitt media (TH media, Difco Labs, Detroit, MI) at 37°C. After the cell density started to increase (Klett about 26-28), cell-free supernatant samples were taken at different stages of growth: early growth phase (about ten minutes), mid-log phase (about 60 minutes) and stationary phase (about 160 minutes).

Further, strain Challis cells cultured as a lawn on SBA plates were scraped from the agar plate surface, incubated for one hour at room temperature in TH media (one scraped plate per 10 ml TH media), and the cells were then removed from the media by centrifugation. In addition, following removal of the cultured cells, the scraped SBA plates were incubated for one hour in 10 ml TH media, and residual cells were removed from the media by centrifugation (10,000 x g for 10 minutes).

In addition, clarified whole saliva that was collected from volunteers and refrigerated at about 0°C was diluted (1:1, vol/vol) with phosphate buffer (Gibbons and Etherden, Infect. Immun., 36:52-58, 1982) and filter sterilized. Using the above described procedures, the following incubating fluids were made: phosphate buffer (control), TH media (control), saliva/phosphate buffer, early growth phase supernatant, mid-log phase supernatant, SBA scrape cells, SBA plate after scraping.

### EXAMPLE 6 - In Vitro Initiation of Bacterial Growth Using a Composition

Bacterial cell-coated bovine chips were prepared from enamel chips as previously described in Example 1. Custom made chip enclosures for the enamel pieces were fabricated in sterile flat-topped microcentrifuge tubes (1.5 ml., Fischer Scientific, Itasca, IL) in Koflex rubber base (Coe Labs, Chicago, IL). Whole saliva was collected from 2-4 volunteers, refrigerated at about 0°C, clarified by centrifugation at 10,000 x g for 10 minutes, and filter sterilized. The mounted enamel pieces were incubated with sterile saliva for 30 to 45 minutes, the saliva decanted and sterile phosphate buffer was used to rinse 2X unattached saliva.

The saliva-coated enamel pieces were then covered with S. gordonii strain Challis ATCC Deposit No. 35105. Briefly, bacteria were grown overnight in Todd Hewitt media (TH media, Difco Labs, Detroit, MI). The bacteria were taken directly from the incubator, mixed within the culture supernatant, and 1.0 ml of the mixture was placed in a microcentrifuge tube containing a saliva-coated enamel chip. The microcentrifuge tubes containing bacterial culture were spun at 10,000 x g for 10 minutes at room temperature. Following centrifugation, the nonadherent bacteria were decanted from adherent S. gordonii ATCC Deposit No. 35105 cells and the tubes containing the adherent cells were placed in an anaerobic chamber.

The bacteria-coated enamel chips were then incubated in the presence of SMR factor-containing incubating fluids in Example 5, above.

To evaluate bacterial growth the chips were moved to a 24-well flat-bottomed chamber (Corning Glass Works, Corning, NY) for subsequent incubation with bacterial growth inhibiting or encouraging fluids. Twelve enamel chip pieces were inserted into custom fitted enclosures for each experiment. The test materials fit tightly into the rubber base without allowing bacteria to adhere behind or around them.

Growth evaluation consisted of a 2-hour incubation time with various liquids listed in Table 3, below, placed with 12 enamel chips. For each chip, bacterial cell viability (viable cells per mm² surface) was determined as described in Examples 1 and 2.

To determine viable cells, the chips were removed, placed in 1.0 ml pre-reduced anaerobically sterilized diluent (PRAS) in an anaerobic chamber (Coy Anaerobic Chamber, Ann Arbor, Michigan) and sonified for 20 seconds (Kontes Cell Disruptor, Kontes, Vineland, NJ). The plaque samples were serially diluted and plated (Spiral Systems, Inc., Cincinnati, OH) on supplemented blood agar and Mitis-salivarius (Difco Labs, Detroit, MI) agar media.

**TABLE 3**

| Incubatinq Fluid | CFU/mm2 After 2 Hour Incubation | % Based on Buffer |
|---|---|---|
| Phosphate buffer (control) | 16,000 | 100% |
| TH media (control) | 16,800 | 105% |
| Saliva/Phosphate Buffer | 40,000 | 250% |
| Early-log supernatant | 78,000 | 488% |
| Mid-log supernatant | 41,000 | 256% |
| SBA scraped cells | 98,800 | 618% |
| SBA plate after scraping | 34,000 | 213% |

### EXAMPLE 7 - In Vitro Assay for SMR Activity

In order to evaluate SMR activity, another in vitro assay was developed.

Bacteria (0.01-2.5 x 10⁷) were preferably bound to Reacti-Bind™ maleic anhydride activated polystyrene strip plates (Pierce, Rockford, Ill), at a preferred concentration of 1 X 10⁸ ml (200 µl per well or 2 X 10⁷ per well) according to the manufacturer and by centrifugation for 10 min. at 30 - 35°C at 3000 rpm (PR-6000 International). Alternatively, bacteria were centrifuged (in similar concentrations, preferably 2 X 10⁷ per well) onto Immulon (Dynatech Laboratories) 96-well plates or Corning Brand Easy Wash ELISA Strip wells. Preferably, the bacteria are S. gordonii strain Challis ATCC deposit No. 35105 and the plates are 96-well maleic anhydride activated plates. The concentration of bacteria is preferably that amount that covers about 20-40% of the surface area of the well. Following centrifugation, unbound bacteria were removed.

The warmed (35-37°C) SMR containing supernatants and control supernatants or isolated SMR preparations were then added to the wells and incubated for 30 minutes at 37°C. After incubation for 30 minutes, radiolabelled nucleosides were added (see Example 1) and the cells were incubated for another 30 minutes. After the incubation in the presence of the radiolabelled nucleosides, the wells are washed and then added to scintillation vials and counted. From time to time free cells in the SMR radiolabelled incubation fluid were also captured before washing on cellulosic filters after incubation with nucleosides. From time to time, bacteria were removed from plates and were TCA precipitated and then analyzed for the radioactivity in the TCA insoluble material. In most instances, the amount of radioactivity incorporated into TCA insoluble material was the same amount of radioactivity detected in plates without TCA precipitation. From time to time plates were incubated for longer time periods.

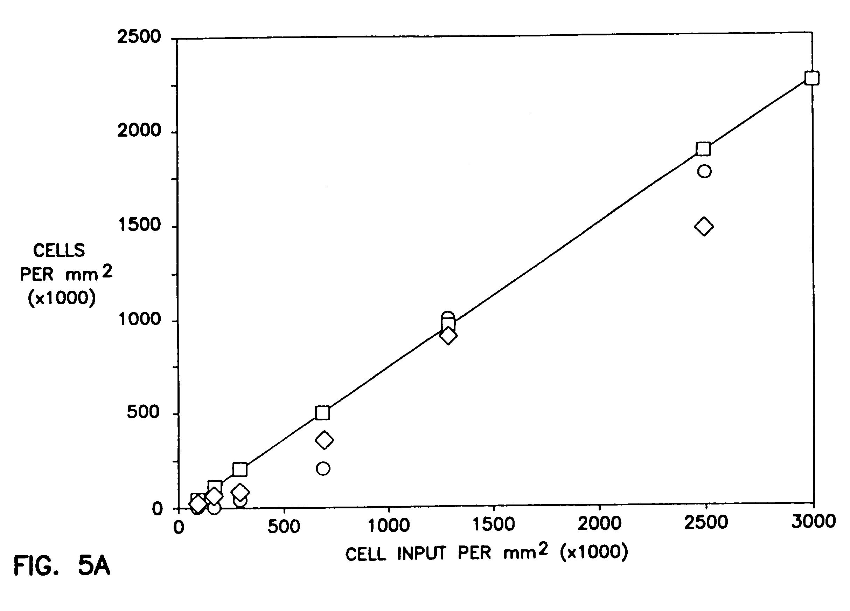

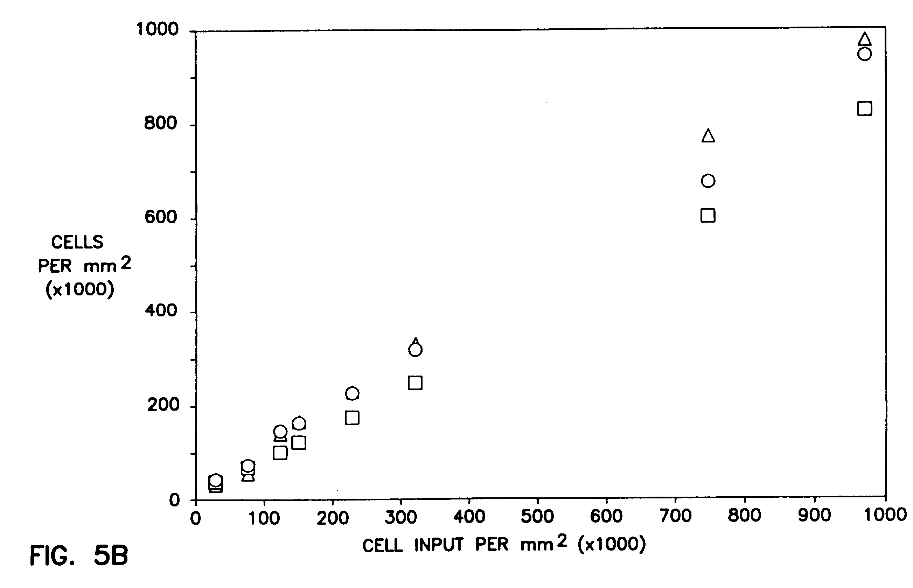

Immobilization of bacteria to plates with maleic anhydride was analyzed as a function of the amount of bacteria added to the wells per mm². Bound bacteria was determined by binding cells prelabeled with 10 µci/ml [methyl-³H]-thymidine. Liljemark and Bloomquist, Infect. Immunol., 34:935-941 (1981). The results are shown in Figure 5A. The results show that the number of bacteria bound/mm² by maleic anhydride increased linearly with the number of bacteria added to the well. Binding of bacteria to maleic anhydride, Immulon and Easy Wash strip plates was also done as described. The results show the number of bacteria increased linearly with the number of bacteria added to the well, Figure 5B.

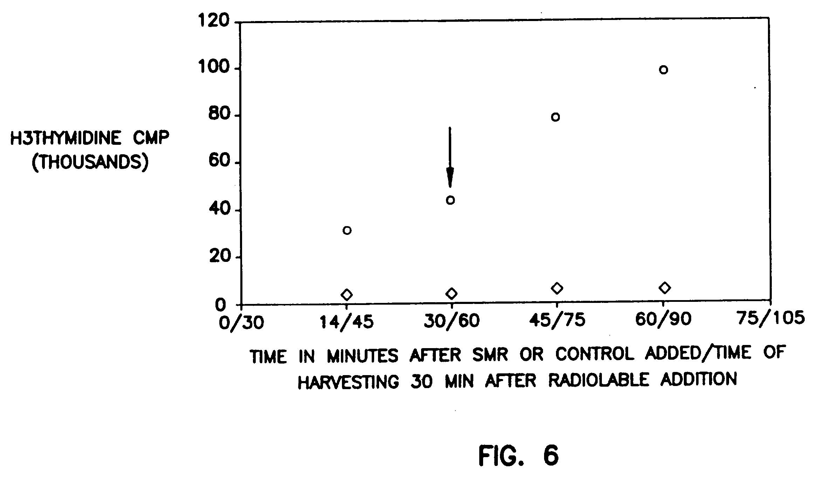

Incorporation of [methyl-H³]-thymidine over time into S. gordonii Challis cells bound to maleic anhydride in the presence or absence of isolated SMR factor (see Example 8) is shown in Figure 6. The results show that after 60 minutes of incubation with purified SMR preparation, there is about a 15-fold increase in the incorporation of [methyl-H³]-thymidine in cells exposed to the SMR factor compared with control cells (see arrow).

The incorporation of [methyl-H³]-thymidine into bound S. gordonii Challis exposed to SMR factor in vitro was compared with incorporation of [methyl-H³]-thymidine to in vivo adherent plaque population, as described in Example 1, as a function of density. The results are shown in Table 1 in Example 1.

The results indicate that the incorporation of [methyl-H³]-thymidine into plaque bacteria in vivo at a density of about 2,500,000 to 3,900,000 [2010^{6.4} - 10^{6.5}] is about 20 x 10⁻⁴ cpm/bacteria and incorporation into bacteria adherent to maleic anhydride at a density of about 630,000 bacteria/mm² is 80 x 10⁻⁴ cpm/bacteria. The in vivo plaque bacteria at a density of 200,000 - 630,000 compared with in vitro bacteria at a density of 625,000 without SMR factor incorporated [methyl-H³]-thymidine to about the same extent.

### EXAMPLE 8 - Isolation and Characterization of SMR Factor

The SMR factor can be obtained from growing cultures of streptococcal strains as well as E. coli ATCC No. 55535. The preferred producer strain is S. gordonii Challis ATCC No. 35105.

Methods for obtaining SMR from bacterial cultures are described in Example 5. In an alternative version, the following methods for producing SMR can be employed. Inoculum was prepared by growing bacteria in Todd-Hewitt media (TH) for 16-24 hours at 37°C [anaerobically for streptococci, anaerobically or aerobically for E. coli]. A mixture of 100 µl inoculum and 2 ml TH media was prepared and placed on top of agar media plates. Preferably, the agar media was supplemented blood agar, as described in Example 1. Alternatively, TH agar or RMP1-1640 broth (JRH Biologicals Inc., Lenexa, Kansas) plus 1.5% agar (Difco) were used. Preferably, TH was mixed with the inoculum. Alternatively, RPMI-1640 or FMC broth (Terlecky et al., Infect. Immunol., 11:649-655 (1975); Terlecky and Shockman, Infect. Immunol., 11:656-664) (1975)) were used. The plates with a media plus bacteria-agar interface were incubated upright for 16-24 hours anaerobically at 37°C.

After incubation, 5 ml of media was added to each plate. Preferably, the media was TH or FMC broth; alternately RPMI-1640 was used. The cells were scraped from the wetted plates and mixed with the added liquid. Following about 20 minutes at room temperature, the liquid mixture was centrifuged at 10,000 x g for 10 minutes to produce complete media SMR factor(s). Following centrifugation, the cellfree supernatant was processed by centrifugation in Centriprep concentrator (Amicon Inc., Beverly, MA) with an exclusion of about 3000 molecular weight, following manufacturer's instructions. This produced SMR factors containing entities of less than about 3000 molecular weight.

Using the same methods (incubation, scraped plates, centrifugation and centriprep), SMR was produced from E. coli ATCC No. 55535. SMR can also be produced from other strains of bacteria.

Control preparations were made by the same methods (incubation, scraped plates, centrifugation and centriprep) without bacterial inoculum to produce control fluids, named according to the media used to inoculate and/or added to the plate following incubation, for example, TH control.

The ability of SMR factors from different sources, and in different media, to stimulate microbial growth of three different bacterial strains was tested by determining incorporation of [methyl-H³]-thymidine in bacteria as described previously. The results are shown in Table 4.

**TABLE 4**

| **Incorporation of [methyl-**^{**3**}**H]-Thymidine cpm X 10**^{**-4**}**/Bacteria** | | | | |
|---|---|---|---|---|
| | | Immobilized Strain | | |
| Bacterial Source of SMR Factor | Media Used To Produce SMR | S. gordonii Challis^{a} | S. gordonii S7^{d} | E. coli^{b} |
| S. gordonii Challis^{a} | TH | 80 | 1.1 | 39 |
| | RPMI-1640 | 48 | ND | ND |
| | FMC | 58 | ND | ND |
| E. coli^{b} | TH | 41 | 1.4 | 14 |
| Control^{c} | TH | 5.8 | 1.4 | 6.5 |
| | RPMI-1640 | 10 | ND | ND |
| | FMC | 12 | ND | ND |

| | | | | |
|---|---|---|---|---|
| ^{a} = S. gordonii Challis ATCC Deposit No. 35105. | | | | |
| ^{b} = E. coli strain 149, ATCC Deposit No. 55535. | | | | |
| ^{c} = Control incubated and isolated as for SMR factor(s). | | | | |
| ^{d} = S. gordonii strain S7 (SK8). ND = No data. | | | | |

The results indicate SMR from S. gordonii Challis ATCC No. 35105 or E. coli ATCC No. 55535 produced in three broths was effective in stimulating microbial growth of either Challis or E. coli. The results of these studies show both gram negative and gram positive bacteria produce SMR factor(s) and both gram positive and gram negative bacteria can respond to SMR factor from either source. The SMR factor(s) from Challis had the most activity.

The complete media SMR factor produced in TH broth prior to 3000 molecular weight membrane exclusion were treated with various agents and exposed to different conditions and then analyzed for SMR activity. Control TH media was also treated or exposed similarly. The complete media SMR was treated with proteinase K, trypsin, DNAase, and RNAase (Sigma) under standard conditions for each enzyme. The enzyme treated SMR retained biological activity. The complete media SMR and isolated SMR factors have been boiled, frozen and autoclaved, and retained biological activity. These results indicate SMR in TH media can be characterized as resistant to trypsin, proteinase K, DNAase, RNAase; and are resistant to heat and freezing.

### EXAMPLE 9 - Ability of SMR Factor to Enhance Incorporation

### of [methyl-H³]-Thymidine into Different Species of Bacteria

Different species and genera of bacteria were immobilized on 96-well maleic anhydride plates to determine if the isolated SMR factor could stimulate microbial growth as measured by uptake of [methyl-H³]-thymidine. The bacteria were incubated with SMR factor or control preparations as previously described (Example 7). The SMR factor was obtained from S. gordonii Challis strains ATCC Deposit No. 35105 and isolated as described in Example 8. The results are shown in Table 5.

The fold increase in [methyl-H³]-thymidine incorporation was determined by dividing the cpm/well for these bacteria incubated with SMR factor by the cpm/well for the same bacteria strain incubated with TH control.

**TABLE 5**

| Bacteria | Fold Increase of Incorporation of ³H Thymidine over Control |
|---|---|
| Streptococcus gordonii S7 | 0.8 |
| S. gordonii Blackburn | 12.8 |
| S. gordonii 12NA | 7.6 |
| Streptococcus parasanguis FW213 | 8.5 |
| Streptococcus mutans IB1600 | 18.2 |
| Streptococcus sanguis 133-79 | 1.6 |
| Streptococcus pneumoniae | 7.4 |
| Streptococcus pyogenes | 10.5 |
| Streptococcus faecalis ATCC 29212 | 9.4 |
| S. faecalis ATCC 19433 | 7.5 |
| Staphlococcus aureus | 7.4 |
| Staphlococcus epidermidis | 7.4 |
| Psuedomonas aeruginosa | 11.1 |
| E. coli ATCC Deposit No. 55535 | 9.1 |
| E. coli Strain TL | 6.3 |
| Haemophilus parainfluenza HP-28 | 7.4 |

The results indicate that the SMR factor from S. gordonii Challis strain ATCC Deposit No. 35105 can stimulate microbial growth of both gram positive and gram negative strains of bacteria.

### EXAMPLE 10 - Preparation of Genetically Engineered S. gordonii Challis Strain with a Reporter Gene for Signalling Microbial Growth

A bacterial strain expressing amylase demonstrates a stimulation of growth by the SMR growth factor. The amylase gene has been inserted into a 16S ribosomal RNA gene (16S rDNA) of the S. gordonii strain Challis ATCC Deposit No. 35105, because it is likely that transcription of ribosomal genes would increase significantly during the burst of growth induced by SMR. Insertion of other reporter genes into 16S rDNA, or insertions of reporter genes into other SMR-inducible bacterial operons, can similarly facilitate the development of improved SMR assays. S. gordonii Challis strain ATTC Deposit No. 35105 does not have an endogenous amylase gene or enzyme activity. Preferably, reporter genes and gene products are selected that are not present in the host bacterial cell.

The bacterial strains to be used in the assays were constructed as follows. Polymerase chain reaction (PCR) was used to enzymatically amplify an internal fragment of 16S rDNA of streptococcal strain Challis and available as ATCC No. 35105. Genomic DNA was purified from this strain as described by Bensing et al., J. Bacteriol., 175:7421 (1993). By inspection of the conserved sequences of streptococcal rDNAs as deposited in the Ribosomal DNA data base, Ribosomal Data Base, Dept. of Microbiol., Univ. of Illinois at Urbana/Champaign, a 20bp and an 18bp sequence was chosen within predicted highly conserved regions of the gene. Synthetic oligonucleotide primers were made using an Applied Biosystems 391 automated sequencer according to manufacturers instructions. These sequences, 5'-TCAGGAATTCGGCTAACTACGTGCCAGCAG-3' (SEQ ID NO:1) and 5'-GAGCGGATCCCCCGGGAACGTATTCAGC-3' (SEQ ID NO:2), corresponding approximately to the predicted sequence of bases 554-573 and the complement of bases 1462-1479 of the S. gordonii Challis ATCC No. 35105 16S rRNA gene (with an EcoRI restriction cleavage site added to the 5' end and a BamHI site added to the 3' end) were used as primers for PCR. The PCR reaction was carried out with a template consisting of 75 ng of S. gordonii Challis ATCC No. 35105 chromosomal DNA, 1 µM of each primer, 300 µM each dNTP, with 10% dimethylsulfoxide, and 4 mm MgCl₂. F.M. Ausubel et al., Current Protocol in Molecular Biology, New York, Greene Publishing Associates and Wiley-Interscience, (1987).

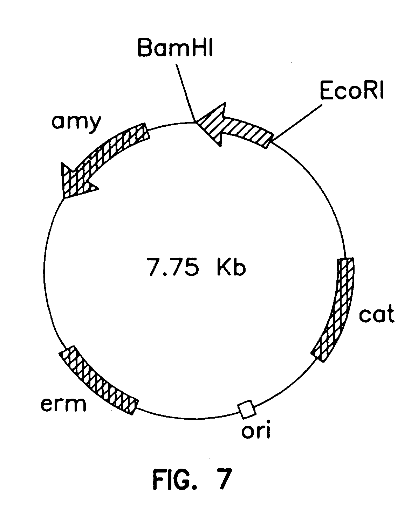

The amplification was carried out in a Hybaid thermal cycler using Vent DNA polymerase from New England Biolabs. Following PCR, an amplified DNA fragment of the predicted size ( 900bp) was detected as the primary product of the PCR by agarose gel electrophoresis. This fragment was cut with EcoRI and BamHI (because of an internal EcoRI site in the amplified DNA about 100 bp was deleted from the 5' end of the fragment), ligated into the site of the vector pRQ200 to generate the plasmid pMSP16Samy as shown in Figure 7 using standard recombinant DNA methodology. The plasmid pRQ200 was constructed as described by Lane et al., cited supra. and was obtained from Dr. Yasbin at University of Maryland, Baltimore, MD. The plasmid was transformed into E. coli DHSαMCR (BRL/Gibco). Transformants were selected for erythromycin resistance.

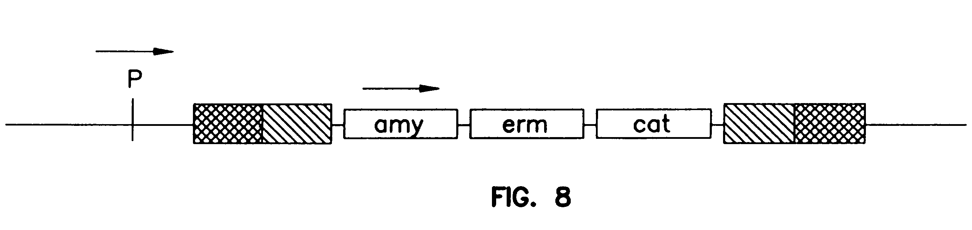

Note the position of the rDNA sequence adjacent to the promoterless amylase gene in Figure 7. pMSP16Samy was then added to competent S. gordonii Challis ATCC No. 35105 cells to allow for uptake of the pMSP16Samy by competent cell transformation as described by Macrina et al., Gene, 19:345 (1982). As described by Lane et al., cited supra., the plasmid cannot replicate autonomously in and, thus, transformants expressing the pRQ200 erythromycin resistance marker can only arise by the integration of the plasmid into the chromosome via the recombination between the cloned rDNA sequences and those in the genome. The predicted structure of such a recombinant is shown in Figure 8. Because there are believed to be about seven repeats of rDNA genes in bacteria such as S. gordonii Challis ATCC No. 35105, the integration could occur in different locations in different transformants. Therefore about 20 different transformants were picked, purified and tested for amylase activity. In the assays described below, two strains obtained by this procedure MSP812 and MSP818 (ATCC 69528 and ATCC 69529, respectively) expressed amylase and have been chosen for use in this assay.

Strain MSP812 is a S. gordonii Challis ATTC Deposit No. 35105 strain that produces amylase activity during microbial growth and was deposited with the ATCC, Rockville, MD on January 6, 1994 and given Accession No. 69528. Strain MSP818 is a S. gordonii Challis ATCC Deposit No. 35105 strain that expresses amylase activity as a function of microbial growth and was deposited with the ATCC in Rockville, MD on January 6, 1994 and given Accession No. 69529. Other relevant characteristics of these organisms are that they are gram positive cocci growing in chains and resistant to erythromycin.

### EXAMPLE 11 - In vitro Assay for Microbial Growth in Bacterial Strains Engineered with a Reporter Gene for Growth

Bacterial strains constructed as described in Example 10 were used to assay the SMR factor. Amylase production in bacteria was monitored in bacteria, incubated in the presence or absence of the isolated SMR factor, using a starch plate amylase assay. Bacterial cultures were grown anaerobically overnight at 37°C (about 10⁹ cells/ml) of S. gordonii Challis ATCC No. 35105 (negative control), S. gordonii Challis ATCC No. 65928, or S. gordonii Challis ATCC No. 65929 in Todd Hewitt media (TH, Difco). The bacteria are harvested by centrifugation, washed with an equal volume of TH, and resuspended in an equal volume of TH. These resuspended bacteria were diluted 10-Fold into TH containing either the SMR factor preparation to be tested, or control extracts. Five µl of these suspensions are inoculated onto the surface of Todd Hewitt agar plates containing 0.2% starch. A series of replicate plates containing the same inocula are prepared and incubated at 37°C in an anaerobic chamber. At thirty minute intervals, plates are removed from the incubator and flooded with Lugol's solution (0.5% iodine, 1% potassium iodide).

The results of the starch plate assay are shown in Table 6. Regions of the plate where amylase activity has degraded the starch a clear zone is apparent against a dark background around the areas where the bacteria have grown. In cases where SMR factor activity is present there will be one or two time points (usually 2-3 hr.) where the SMR-treated bacteria have produced more amylase, and thus show a more intense "halo" of clearing around the region of bacterial growth than the control-treated bacteria grown on the same plate. The data are depicted in Table 6.

**TABLE 6**

| Strain Clearing zone at: | 1h | 1.5h | 2h | 2.5h | 3h | 4h |
|---|---|---|---|---|---|---|
| Challis ATCC Deposit No. 35105 | 0 | 0 | 0 | 0 | 0 | 0 |
| | | | | | | |
| pMSP812 (control) | 0 | 0 | 0 | 0 | 1 | 3 |
| | | | | | | |
| pMSP812 (START) ATCC Deposit No. 69528 | 0 | 0 | 2 | 3 | 3 | 3 |
| | | | | | | |
| pMSP818 (control) | 0 | 0 | 0 | 1 | 2 | 3 |
| | | | | | | |
| pMSP818 (START) ATCC Deposit No. 69529 | 0 | 1 | 2 | 3 | 3 | 3 |

| | | | | | | |
|---|---|---|---|---|---|---|
| 0 - no zone of clearing around the bacterial colony | | | | | | |
| 1 - zone of clearing surrounding the rim of the bacterial colony | | | | | | |
| 2 - observable zone of clearing less than 1 mm. | | | | | | |
| 3 - well defined zone of clearing around the bacterial colony. | | | | | | |

Transformed and nontransformed Challis strains were also tested for the ability respond to the SMR factor in the in vitro assay. Briefly, S. gordonii Challis ATCC Deposit No. 35105, S. gordonii Challis ATCC Deposit No. 65928, or S. gordonii Challis ATCC Deposit No. 65929 were bound onto maleic anhydride plates, as described in Example 7.

**TABLE 7**

| **Incorporation of [methyl-**^{**3**}**H]-Thymidine cpm X 10**^{**-4**}**/Bacteria** | | |
|---|---|---|
| Bacterial Strain | SMR | TH (Control) |
| Challis strain ATCC No. 35105 | 80 | 5 |
| | | |
| Challis strain MSP812 ATCC No. 69528 | 107 | 4 |
| | | |
| Challis strain MSP18 ATCC No. 69529 | 147 | 5 |

The results show that the transformed strains responded to the SMR factor similarly to the nontransformed strain. While not meant to limit the invention, the transformed strains have utility for measuring microbial growth in the described amylase assay whether or not they respond to the SMR factor.

## Claims

1. A composition comprising a start microbial replication factor, wherein the start microbial replication factor is produced by bacteria and has a molecular weight of less than about 3000 as measured by size exclusion membrane filtration.

2. A composition of claim 1 wherein the start microbial replication factor is resistant to autoclaving.

3. The composition of claim 1 wherein the start microbial replication factor is isolated from sanguis streptococcus.

4. The composition of claim 1 wherein the start microbial replication factor is isolated from S. gordonii Challis strain ATCC Deposit No. 35105.

5. A composition according to claim 1 wherein the start microbial replication factor is isolated from E. coli 149, strain ATCC Deposit No. 55535.

6. The composition according to claim 1 wherein the start microbial replication factor stimulates growth of bacteria.

7. The composition of claim 1 further comprising components elected from the group consisting of cations, anions, enzymes, transport polypeptides, coenzymes, cofactors and polypeptides.

8. The composition of claim 1 wherein the start microbial replication factor stimulates growth of bacteria at a population density below a density associated with an enhancement or stimulation of the proportion of actively growing cells to viable cells in a population.

9. The composition of claim 1 wherein the start microbial replication factor stimulates or enhances the growth of bacterial cells in a population to achieve a ratio of growing cells to viable cells in the population of about 5 X 10⁻⁴:1 to 20 X 10⁻²:1.

10. A composition of claim 1 wherein the start microbial replication factor is isolated from bacteria growing on a surface.

11. A composition of claim 2, wherein the start microbial replication factor is isolated from bacteria growing in suspension.

12. A method of increasing the efficacy of an antimicrobial agent comprising the step of contacting a bacteria with the antimicrobial agent in the presence of the start microbial replication factor of claim 1 in order to initiate active growth of the bacteria.

13. A method according to claim 12, wherein the microbial agent is specific for the bacteria.

14. A method for determining whether an agent inhibits or enhances the activity of a start microbial replication factor comprising:
a) contacting a population of bacteria with an agent suspected of enhancing or inhibiting a start microbial replication factor and the start microbial replication factor of claim 1 to form a treated population,
b) determining a proportion of growing bacteria in the treated population; and
c) determining an enhancement or inhibition of a start microbial replication factor by comparing the proportion of growing bacteria in the treated population to a proportion of growing cells in a bacterial population contacted with the start microbial replication factor.

15. A method according to claim 14 wherein the amount of growth is determined by measuring the amount of incorporation of a radiolabelled nucleotide per bacteria.

16. A method according to claim 14 wherein the bacteria is S. gordonii Challis ATCC Deposit No. 35105.

17. A method according to claim 14 wherein the bacteria are bound to a surface.

18. A method according to claim 14 wherein the agent suspected of inhibiting or enhancing start microbial replication factor is bound to a surface.

19. A method for measuring the activity of a start microbial replication factor on bacteria comprising:
a) contacting a population of bacteria with the start microbial replication factor of claim 1 to form a treated population; and
c) determining the amount of stimulation of microbial growth by comparing microbial growth in the treated population to microbial growth in an untreated population of bacteria.

20. A method according to claim 19 wherein microbial growth is determined by measuring the amount of incorporation of a radiolabelled nucleotide per bacteria.

21. A method according to claim 19 wherein the population of bacteria is a biologically pure population of S. gordonii Challis ATCC Deposit No. 35105.

22. A method according to claim 19 wherein the population of bacteria is bound to a surface.

23. A method of stimulating microbial growth comprising: contacting a population with an amount of a start microbial replication factor of claim 1 effective to increase the proportion of actively growing cells to viable cells in the population of bacteria.

24. A method according to claim 23, wherein the proportion of actively growing cells to viable cells in the population is increased to a ratio of about 5 x 10⁻⁴:1 to 20 x 10⁻²:1.

25. A method according to claim 22, wherein the population of bacteria are bound to a surface.

26. A method according to claim 23, wherein the population of bacteria are in suspension.

27. A method of predicting when bacteria adhered to a surface will exhibit active growth comprising the steps of
a) determining a first population density of viable bacteria on the surface,
b) comparing the first population density with a second population density having a stimulated or enhanced proportion of actively growing bacteria to viable bacteria in the second population, and
c) predicting active bacterial growth when the first population density is greater than the second population density.

28. The method of claim 27 wherein the density of (b) is between about 400,000 bacteria/mm² to about 6,300,000 bacteria/mm².

29. The method of claim 27 wherein the growth of bacteria occurs on a surface susceptible to bacterial infestation.

30. The method of claim 27 wherein the growth of bacteria occurs on the surface of a dental implant.

31. The method of claim 27 wherein the growth of bacteria occurs on an implanted biomaterial.

32. A method to predict active growth of bacteria on a surface comprising the steps of:
a) determining a population density of viable growing bacteria on the surface,
b) determining a population of actively growing bacteria on the surface,
c) computing a ratio of actively growing bacteria to viable bacteria on the surface, and
d) predicting active bacterial growth on the surface when the ratio of step c) is in the range of about 5 X 10⁻⁴:1 to 20 X 10⁻²:1.

33. The method of claim 32 wherein the population of actively growing bacteria is determined by adding an excess of a labeled nucleoside to the bacteria for a time less than the minimum cell division time of the bacteria, growing the bacteria for an amount of time sufficient to allow bacterial incorporation of the labeled nucleotide and, identifying the amount of labeled nucleotide incorporated into the DNA of the bacteria.

34. The method of claim 33 wherein the labeled nucleoside is selected from the group consisting of radiolabelled purines and pyrimidines.

35. The method of claim 33 wherein the labeled nucleoside is selected from the group consisting of [methyl-³H]-thymidine and [C¹⁴]-adenine and mixtures thereof.

36. The method of claim 32 wherein the ratio of step c) is in the range between about 10 x 10⁻⁴:1 to 40 x 10⁻⁴:1.

37. A method of inhibiting bacterial growth on a surface comprising the step of
a) controlling the population of bacteria on the surface by maintaining the population density of the bacteria below a population density associated with an enhancement or stimulation of the proportion of growing cells to viable cells in the population.

38. The method of claim 37 wherein the population density of the bacteria on the surface is controlled by limiting the contact of adherence promoting agents with the bacteria.

39. The method of claim 38 wherein the adherence promoting agent is selected from the group consisting of polypeptides, glycoproteins and carbohydrates.

40. The method of claim 37 wherein the population density of the bacteria on the surface is controlled by modifying the hydrophobicity of the surface with a adherence suppressing agent.

41. The method of claim 40 wherein the adherence suppressing agent is sodium octadecyl sulfate.

42. The method of claim 37 wherein the population density of the bacteria on the surface is controlled by selecting a material having an mean dynamic contact advancing angle greater than about 60 degrees and a mean dynamic contact receding angle greater than about 20 degrees.

43. The method of claim 42 wherein the mean dynamic contact advancing angle is greater than about 90 degrees.

44. The method of claim 37 wherein bacterial growth occurs in the presence of an aqueous fluid selected from the group consisting of body fluids, sera, carbohydrate-containing fluids, polypeptide-containing fluids, glycoprotein-containing fluids, or water.

45. A genetic cassette, comprising:
a) a first DNA sequence coding for a bacterial reporter gene linked to the 3' end of
b) a second DNA sequence that hybridizes to a gene or portion thereof whose expression is coordinately regulated with cell growth and is located in the bacterial chromosome to form the genetic cassette, wherein the second DNA sequence provides for recombination and integration of the genetic cassette or a portion thereof into the gene in the bacterial chromosome.

46. A genetic cassette according to claim 45 wherein the first DNA sequence codes for a bacterial amylase.

47. An genetic cassette of claim 45 wherein the second DNA sequence codes for a portion of a streptococcal 16S ribosomal RNA gene.

48. A genetic cassette according to claim 45 further comprising a selectable marker gene linked to the first and second DNA sequences.

49. A plasmid having a genetic cassette of claim 45.

50. A bacterial strain having a genetic cassette or a portion thereof of claim 45 stably integrated into the bacterial chromosome at a location under the control of a promoter whose expression is coordinately regulated with cell growth.

51. A bacterial strain according to claim 50 wherein the bacterial host cell is S. gordonii Challis strain MSP818 ATCC No. 69529.

52. A bacterial strain according to claim 50 wherein the bacterial host cell is S. gordonii Challis strain MSP812 ATCC Deposit No. 69528.

53. A bacterial strain having a genetic cassette or a portion thereof of claim 45, wherein the bacterial strain does not have an endogenous DNA sequence coding for the bacterial reporter gene product.

54. A method for measuring activity of a start microbial replication factor comprising:
a) providing a population of bacteria, wherein the population of bacteria has a stably integrated genetic cassette or a portion thereof under control of a promoter whose expression is coordinately regulated with cell growth wherein the cassette comprises:
(i) a first DNA sequence coding for a bacterial reporter gene product, linked to the 3' end of
ii) a second DNA sequence hybridizing to a gene or portion thereof whose expression is coordinately regulation with cell growth and is located in the bacterial chromosome to form the genetic cassette, wherein the second DNA sequence has sufficient sequence identity to the gene or portion thereof to provide for recombination and integration of the genetic cassette or a portion thereof into the gene in the bacterial chromosome,
b) contacting the population of bacteria with a start microbial replication factor to form a treated population; and
c) determining the amount of microbial growth by measuring the amount of reporter gene product produced per unit time per cell in the treated population compared to the amount of reporter gene product per unit time per cell in an untreated population.

55. A method of measuring microbial growth comprising:
a) providing a population of bacteria, wherein the population of bacteria has a stably integrated genetic cassette or a portion thereof under control of a promoter whose expression is coordinately regulated with the rate of cell growth, wherein the cassette comprises:
(i) a first DNA sequence coding for a bacterial reporter gene product, linked to the 3' end of
ii) a second DNA sequence hybridizing to a gene or portion thereof whose expression is coordinately regulated with cell growth and is located in the bacterial chromosome, to form the genetic cassette, wherein the second DNA sequence provides for recombination and integration of the genetic cassette or a portion thereof into the gene in the bacterial chromosome; and
b) determining the amount of reporter gene product in the population.
